# EUROPÄISCHE PATENTSCHRIFT

(11) **EP 3 216 797 B1**
(45) Veröffentlichungstag und Bekanntmachung des Hinweises auf die Patenterteilung: **03.03.2021**
(21) Anmeldenummer: 17164847.0
(22) Anmeldetag: 13.10.2010
(51) Int. Cl.: C07F 15/00, H01L 51/00, H05B 33/00

(54) **DINUKLEARE PLATIN-CARBEN-KOMPLEXE UND DEREN VERWENDUNG IN OLEDS**
DINUCLEAR PLATINUM-CARBENE COMPLEXES AND THE USE THEREOF IN OLEDS
COMPLEXE DE CARBÈNE/PLATINE DINUCLÉAIRE ET SON UTILISATION DANS DES DELO

(30) Priorität: 14.10.2009 US 251320 P
(43) Veröffentlichungstag der Anmeldung: 13.09.2017
(62) Teilanmeldung aus: 10773002.0
(73) Patentinhaber: UDC Ireland Limited, Ballycoolin, Dublin 15 (IE)
(72) Erfinder: MOLT, Oliver, 69469 Weinheim (DE); LENNARTZ, Christian, 67105 Schifferstadt (DE); WAGENBLAST, Gerhard, 67157 Wachenheim (DE); STRASSNER, Thomas, 01156 Dresden (DE); UNGER, Yvonne, 01159 Dresden (DE)
(74) Vertreter: Hansen, Norbert

(56) Entgegenhaltungen:
- WO-A1-2007/088093
- WO-A2-2006/056418
- WO-A2-2008/141637
- S.-W. LAI ET AL.: "Synthesis of Organoplatinum Oligomers by Employing N-Donor Bridges with Predesigned Geometry: Structural and Photophysical Properties of Luminescent Cyclometallated Platinum(II) Macrocycles", ORGANOMETALLICS, Bd. 18, Nr. 20, 28. August 1999 (1999-08-28), Seiten 3991-3997, XP002615229,
- N. STYLIANIDES ET AL.: "Cyclometalated and Alkoxyphenyl-Substituted Palladium Imidazolin-2-ylidene Complexes. Synthetic, Structural, and Catalytic Studies", ORGANOMETALLICS, Bd. 26, Nr. 23, 2. Oktober 2007 (2007-10-02), Seiten 5627-5635, XP002615230,

## Beschreibung

Die vorliegende Erfindung betrifft dinukleare Pt-Carben-Komplexe, organische Leuchtdioden (OLEDs) enthaltend wenigstens einen solchen dinuklearen Pt-Carben-Komplex, Licht-emittierende Schichten, enthaltend wenigstens einen solchen dinuklearen Pt-Carben-Komplex, eine Vorrichtung wie beispielsweise stationäre oder mobile Bildschirme oder Beleuchtungsmittel, enthaltend ein entsprechendes OLED, sowie die Verwendung der erfindungsgemäßen dinuklearen Pt-Carben-Komplexe in OLEDs, beispielsweise als Emitter, Matrixmaterial, Ladungstransportmaterial und/oder Ladungsblocker.

Die organische Elektronik ist ein Teilgebiet der Elektronik, das elektronische Schaltungen verwendet, die Polymere oder kleinere organische Verbindungen enthalten. Anwendungsbereiche der organischen Elektronik sind der Einsatz von Polymeren oder kleineren organischen Verbindungen in organischen Leuchtdioden (OLEDs) oder Licht-Emittierenden Elektrochemischen Zellen (LEEC), die Anwendung in organischen Solarzellen (organische Photovoltaik), sowie in schaltenden Elementen wie organischen Transistoren, z. B. organischen FET und organischen TFT.

Durch den Einsatz geeigneter neuartiger organischer Materialien können somit verschiedene neuartige Bauelemente, die auf organischer Elektronik basieren, wie Displays, Sensoren, Transistoren, Speicher oder Photovoltaik-Zellen bereitgestellt werden. Dadurch ist die Entwicklung von Bauteilen für neue Anwendungen, die dünn, leicht, flexibel und zu niedrigen Kosten herstellbar sind, möglich.

Ein bevorzugter Anwendungsbereich gemäß der vorliegenden Erfindung ist der Einsatz der erfindungsgemäßen Verbindungen in organischen Leuchtdioden.

In organischen Leuchtdioden (OLED) wird die Eigenschaft von Materialien ausgenutzt, Licht zu emittieren, wenn diese Materialien durch elektrischen Strom angeregt werden. OLEDs sind insbesondere interessant als Alternative zu Kathodenstrahlröhren und Flüssigkristalldisplays zur Herstellung von Flachbildschirmen. Aufgrund der sehr kompakten Bauweise und des intrinsisch niedrigen Stromverbrauchs eignet sich die Vorrichtung enthaltend OLEDs insbesondere für mobile Anwendungen, z. B. für Anwendungen in Mobiltelefonen, Laptops usw., sowie zur Beleuchtung.

Die Grundprinzipien der Funktionsweise von OLEDs sowie geeignete Aufbauten (Schichten) von OLEDs sind z. B. in WO 2005/113704 und der darin zitierten Literatur genannt.

Als Licht-emittierende Materialien (Emitter) können neben fluoreszierenden Materialien (Fluoreszenz-Emitter) phosphoreszierende Materialien (Phosphoreszenz-Emitter) eingesetzt werden. Bei den Phosphoreszenz-Emittern handelt es sich üblicherweise um metallorganische Komplexe, die im Gegensatz zu den Fluoreszenz-Emittern, die eine Singulett-Emission zeigen, eine Triplett-Emission zeigen (M. A. Baldo et al., Appl. Phys. Lett. 1999, 75, 4 bis 6). Aus quantenmechanischen Gründen ist bei Verwendung der Phosphoreszenz-Emitter eine bis zu vierfache Quanten-, Energie- und Leistungseffizienz möglich.

Von besonderem Interesse sind organische Leuchtdioden mit langer operativer Lebensdauer, guter Effizienz, hoher Stabilität gegenüber Temperaturbelastungen sowie einer niedrigen Einsatz- und Betriebsspannung.

Aus dem Stand der Technik sind verschiedene chemische Verbindungen bekannt, die verschiedene Aufgaben innerhalb einer OLED wahrnehmen können.

WO 2004/093210 A1 offenbart organische Leuchtdioden (OLEDs), die dinukleare Metall-Verbindungen als Emittermaterialien aufweisen. Es werden Leuchtdioden offenbart, die eine Anode, eine Kathode und eine emittierende Schicht zwischen Anode und Kathode aufweisen. Die emittierende Schicht umfasst ein Emittermaterial, welches mehr als ein Metall-Zentrum aufweist. Die dinuklearen Verbindungen gemäß WO 2004/093210 umfassen bevorzugt zwei Metall-Zentren, wobei jedes Metallzentrum an wenigstens einen verbrückenden Liganden angebunden ist. Geeignete verbrückende Liganden gemäß diesem Dokument sind fünf- oder sechsgliedrige Ringe, welche Stickstoff als Heteroatom aufweisen. Des Weiteren wird Pyrazol als verbrückender Ligand genannt. Neben den verbrückenden Liganden enthalten die dinuklearen Komplexe gemäß diesem Dokument weitere Liganden, wobei keine Carben-Liganden in WO 2004/093210 offenbart werden.

T. Koshijama et al., Chem. Lett. Vol. 33, 10, 2004, 1386 -1387 offenbaren cyclometallierte dinukleare Platin-Komplexe, welche mittels Pyridin-2-thiolat-Ionen verbrückt sind. Diese Komplexe weisen bei Anlegen einer elektrischen Spannung eine Lumineszenz auf. Das genannte Dokument offenbart keine dinuklearen Platin-Komplexe, welche verbrückende Pyrazol-Einheiten bzw. Liganden aufweisen, die über Carben-Bindungen an die Platin-Atome angebunden sind.

Saito et al., Jpn. J. Appl. Phys. 2005, 44, L500 - L501 offenbaren eine OLED mit einem dinuklearen cyclometallierten Platinkomplex mit Pyridinthiolatbrücke. Das Dokument offenbart keine dinuklearen Platin-Komplexe mit verbrückenden Pyrazol-Einheiten oder Carben-Metall-Bindungen.

Lai et al., Organometallics 1999, 18, 3991 - 3997 offenbaren pyrazolverbrückte cyclometallierte Platinkomplexe und deren Quantenausbeuten, jedoch sind keine Carben-bindungen zum Metall vorhanden.

M. Tanabe et al., Organometallics 2008, 27, 2258 bis 2267, offenbaren dinukleare Platinkomplexe mit verbrückenden Silyl-Liganden. Zum einen werden keine Komplexe offenbart, die über Pyrazol-Einheiten verbrückte Platin-Atome enthalten. Des Weiteren werden keine Pt-Carben-Komplexe offenbart.

WO 2008/141637 A2 offenbart phosphoreszierende Metall-Komplexverbindungen, strahlungsemittierende Bauelemente, die eine solche phosphoreszierende Metallkomplexverbindung aufweisen und ein Verfahren zur Herstellung der genannten Verbindungen. Neben einkernigen Metall-Komplexverbindungen werden auch dinukleare Platin-Komplexe offenbart. Allgemein wird Pyrazol als möglicher Ligand für entsprechende Komplexe offenbart. Es wird jedoch nicht offenbart, dass Pyrazol ein verbrückender Ligand zwischen Platin-Atomen ist, bzw. dass neben den verbrückenden Pyrazol-Liganden weitere Liganden vorliegen, die über Carben-Bindungen an das oder die Metallatom(e) angebunden sind.

B. Ma et al., J. Am. Chem. Soc. 2005, 127, 28 bis 29, offenbaren dinukleare Platin-Komplexe, die Pyrazol-Einheiten als verbrückende Liganden zwischen den beiden Platin-Atomen aufweisen. Die Komplexe gemäß dieser Schrift weisen keine Liganden auf, die über Carben-Bindungen an die Metallzentren angebunden sind.

Qi et al., Chem. Phys. Lett. 2008, 458, 323-328 und Ma et al., Adv. Funct. Mater. 2006, 16, 2438-2446 offenbaren weiterführende Untersuchungen zu den in Ma et al., J. Am. Chem. Soc. 2005, 127, 28 - 29 vorgestellten dinuklearen Komplexen.

S.-Y. Chang et al., Inorg. Chem. 2007, 46, 11202 - 11212 offenbaren blaues Licht emittierende Platin(II)-Kompexe umfassend zum einen Pyridylpyrazol-Liganden, und zum anderen chelatisierende Pyrazol-Liganden. Entsprechende Komplexe, in denen zwei Platin-Kationen mit Pyrazol bzw. Pyrazol-Derivaten verbrückt sind, werden nicht offenbart.

Jain et al., J. Chem. Soc. Dalton Trans. 1993, 3625-3628 offenbaren pyrazolverbrückte Verbindungen. Diese sind jedoch nicht cyclometalliert und es gibt keine Photoluminszenzdaten.

Hill et al., Inorg. Phys. Theor. 1971, 2341 - 2347 offenbaren dinukleare Metallkomplexe, die Palladium- oder Platin-Kationen enthalten. Die beiden Metallkationen sind über Carboxylate von organischen Carbonsäuren verbrückt. Als weitere Liganden enthalten die Komplexe gemäß Hill et al. Diene.

Powell et al., Inorg. Chem. 1972, 11, 1039 - 1048 offenbaren dinukleare Komplexe, die als Metallkationen Palladium oder Platin enthalten. Die beiden Metallkationen sind über Carboxylgruppen miteinander verbrückt. Die genannten Komplexe tragen als weitere Liganden Diphenylphosphin-Gruppen. S.-W. Lai et al., Organometallics 1999, 20, 3991-3997 beschreibt dinukleare Platinkomplexe mit Phenylimidazoleinheiten. WO 2006/056418 A2 beschreibt die Verwendung von Übergangsmetall-Carbon-Komplexen in organischen lichtemittierenden Dioden. WO 2008/141637 A2 beschreibt dinukleare Platinkomplexe mit Pyrazoleinheiten. WO 2007/088093 A1 beschreibt ein Verfahren zur Herstellung von cyclometallierten Übergangsmetall-Carben-Komplexen mit zumindest einem Carbenliganden. N. Stylianides at al., Organometallics 2007, 23, 5627-5635 beschreibt cyclometallierte Alkoxyphenyl substituierte palladium lmidazolin-2-yliden Komplexe.

Die im Stand der, Technik offenbarten dinuklearen Platin-Komplexe weisen den Nachteil auf, dass, sofern diese Komplexe als Emitter in organischen Leuchtdioden eingesetzt werden, nur unzureichende Quantenausbeuten möglich sind. Des Weiteren sind speziell für die Anwendung in organischen Leuchtdioden notwendige Eigenschaften, beispielsweise Effizienz und Stabilität, der im Stand der Technik genannten dinuklearen Platin-Komplexe verbesserungsbedürftig.

Aufgabe der vorliegenden Erfindung ist daher die Bereitstellung von weiteren, für den Einsatz in organischen Leuchtdioden und weiteren Anwendungen in der organischen Elektronik geeigneten Materialien. Insbesondere sollen Phosphoreszenz-Emitter für den Einsatz in OLEDs bereitgestellt werden. Des Weiteren sollen die Materialien zur Bereitstellung von OLEDs geeignet sein, die gute Effizienzen, gute operative Lebensdauern sowie eine hohe Stabilität gegenüber Temperaturbelastung und eine niedrige Einsatz- und Betriebsspannung der OLEDs gewährleisten.

Diese Aufgaben werden erfindungsgemäß gelöst durch einen dinuklearen Pt-Carben-Komplex der allgemeinen Formel (Ia) 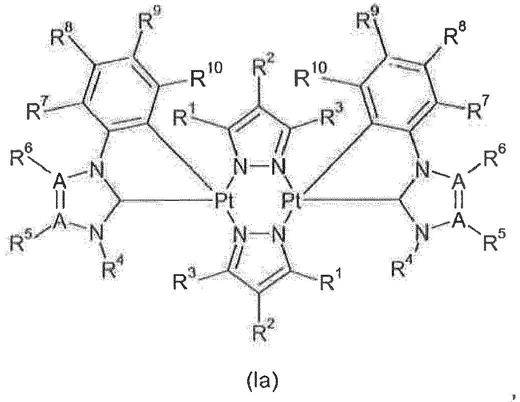 wobei A, R¹, R², R³, R⁴, R⁵, R⁶, R⁷, R⁸ und R⁹ und R¹⁰ die folgenden Bedeutungen aufweisen:
- A: unabhängig voneinander N oder C,
- R¹, R², R³: unabhängig voneinander Wasserstoff, linearer oder verzweigter, gegebenenfalls von wenigstens einem Heteroatom unterbrochener, gegebenenfalls wenigstens eine funktionelle Gruppe tragender Alkylrest mit 1 bis 10 Kohlenstoffatomen, Cycloalkylrest mit 3 bis 10 Kohlenstoffatomen,
- R⁴: unabhängig voneinander, linearer oder verzweigter, gegebenenfalls von wenigstens einem Heteroatom unterbrochener, gegebenenfalls wenigstens eine funktionelle Gruppe tragender Alkylrest mit 1 bis 10 Kohlenstoffatomen, Cycloalkylrest mit 3 bis 10 Kohlenstoffatomen, substituierter oder unsubstituierter Arylrest mit 6 bis 18 Kohlenstoffatomen, substituierter oder unsubstituierter Heteroarylrest mit insgesamt 5 bis 18 Kohlenstoff- und/oder Heteroatomen,
- R⁵, R⁶: unabhängig voneinander freies Elektronenpaar, falls A gleich N, oder, falls A gleich C, Wasserstoff, linearer oder verzweigter, gegebenenfalls von wenigstens einem Heteroatom unterbrochener, gegebenenfalls wenigstens eine funktionelle Gruppe tragender Alkylrest mit 1 bis 10 Kohlenstoffatomen, Cycloalkylrest mit 3 bis 10 Kohlenstoffatomen, substituierter oder unsubstituierter Arylrest mit 6 bis 18 Kohlenstoffatomen, substituierter oder unsubstituierter Heteroarylrest mit insgesamt 5 bis 18 Kohlenstoff- und/oder Heteroatomen,
oder
R⁴ und R⁵ oder R⁵ und R⁶ bilden zusammen mit N und A oder mit A und A einen gegebenenfalls von wenigstens einem weiteren Heteroatom unterbrochenen gesättigten, ungesättigten oder aromatischen Ring mit insgesamt 5 bis 18 Kohlenstoff- und/oder Heteroatomen,
- R⁷, R⁸, R⁹, R¹⁰: unabhängig voneinander Wasserstoff, linearer oder verzweigter, gegebenenfalls von wenigstens einem Heteroatom unterbrochener, gegebenenfalls wenigstens eine funktionelle Gruppe tragender Alkylrest mit 1 bis 10 Kohlenstoffatomen, Cycloalkylrest mit 3 bis 10 Kohlenstoffatomen, substituierter oder unsubstituierter Arylrest mit 6 bis 18 Kohlenstoffatomen, substituierter oder unsubstituierter Heteroarylrest mit insgesamt 5 bis 18 Kohlenstoff- und/oder Heteroatomen, funktionelle Gruppe ausgewählt aus Ether-, Amino-, Thio-, Ester-, Carbonyl-, Nitro- oder Halogen-Gruppe,
oder
R⁷ und R⁸ oder R⁸ und R⁹ oder R⁹ und R¹⁰ bilden zusammen einen gegebenenfalls von wenigstens einem Heteroatom unterbrochenen gesättigten, ungesättigten oder aromatischen, gegebenenfalls substituierten, Ring mit insgesamt 5 bis 18 Kohlenstoff- und/oder Heteroatomen
und/oder
R⁶ und R⁷ bilden zusammen eine gesättigte oder ungesättigte, lineare oder verzweigte, gegebenenfalls Heteroatome, aromatische Einheiten, heteroaromatische Einheiten und/oder funktionelle Gruppen enthaltende Verbrückung mit insgesamt 1 bis 30 Kohlenstoff- und/oder Heteroatomen, an die gegebenenfalls ein substituierter oder unsubstituierter, fünf- bis achtgliedriger, Kohlenstoff- und/oder Heteroatome enthaltender Ring, annelliert ist.

Im Sinne der vorliegenden Erfindung haben die Begriffe Arylrest oder -gruppe, Heteroarylrest oder -gruppe und Alkylrest oder -gruppe, die folgenden Bedeutungen:
Unter einem Arylrest oder -gruppe ist ein Rest mit einem Grundgerüst von 6 bis 30 Kohlenstoffatomen, bevorzugt 6 bis 18 Kohlenstoffatomen zu verstehen, der aus einem aromatischen Ring oder mehreren kondensierten aromatischen Ringen aufgebaut ist. Geeignete Grundgerüste sind zum Beispiel Phenyl, Benzyl, Naphthyl, Anthracenyl oder Phenanthrenyl. Dieses Grundgerüst kann unsubstituiert sein, d. h., dass alle Kohlenstoffatome, die substituierbar sind, Wasserstoffatome tragen, oder an einer, mehreren
oder allen substituierbaren Positionen des Grundgerüsts substituiert sind. Geeignete Substituenten sind zum Beispiel Alkylreste, bevorzugt Alkylreste mit 1 bis 8 Kohlenstoffatomen, besonders bevorzugt Methyl, Ethyl, i-Propyl oder t-Butyl, Arylreste, bevorzugt C₆-Arylreste, die wiederum substituiert oder unsubstituiert sein können, Heteroarylreste, bevorzugt Heteroarylreste, die mindestens ein Stickstoffatom enthalten, besonders bevorzugt Pyridylreste, Alkenylreste, bevorzugt Alkenylreste, die eine Doppelbindung tragen, besonders bevorzugt Alkenylreste mit einer Doppelbindung und 1 bis 8 Kohlenstoffatomen, oder Gruppen mit Donor- oder Akzeptorwirkung. Unter Gruppen mit Donorwirkung sind Gruppen zu verstehen, die einen +I- und/oder +M-Effekt aufweisen, und unter Gruppen mit Akzeptorwirkung sind Gruppen zu verstehen, die einen -I- und/oder -M-Effekt aufweisen. Geeignete Gruppen, mit Donor- oder Akzeptorwirkung sind Halogenreste, bevorzugt F, Cl, Br, besonders bevorzugt F, Alkoxyreste, Aryloxyreste, Carbonylreste, Esterreste, Aminreste, Amidreste, CH₂F-Gruppen, CHF₂-Gruppen, CF₃-Gruppen, CN-Gruppen, Thiogruppen oder SCN-Gruppen. Ganz besonders bevorzugt tragen die Arylreste Substituenten ausgewählt aus der Gruppe bestehend aus Methyl, F, Amin, Thiogruppe und Alkoxy, oder die Arylreste sind unsubstituiert. Bevorzugt ist der Arylrest oder die Arylgruppe ein C₆-Arylrest, der gegebenenfalls mit mindestens einem der vorstehend genannten Substituenten substituiert ist. Besonders bevorzugt weist der C₆-Arylrest keinen, einen, zwei oder drei der vorstehend genannten Substituenten auf. Besonders bevorzugt weisen die erfindungsgemäß vorhandenen Arylreste keine Substituenten auf.

Unter einem Heteroarylrest oder einer Heteroarylgruppe sind Reste zu verstehen, die sich von den vorstehend genannten Arylresten dadurch unterscheiden, dass in dem Grundgerüst der Arylreste mindestens ein Kohlenstoffatom durch ein Heteroatom ersetzt ist. Bevorzugte Heteroatome sind N, O und S. Ganz besonders bevorzugt sind ein oder zwei Kohlenstoffatome des Grundgerüsts der Arylreste durch Heteroatome ersetzt. Insbesondere bevorzugt ist das Grundgerüst ausgewählt aus Pyridyl, Pyrimidyl, Pyrazyl, Triazyl, und fünfgliedrigen Heteroaromaten wie Pyrrol, Furan, Thiophen, Pyrazol, Imidazol, Triazol, Oxazol, Thiazol. Das Grundgerüst kann an keiner, einer, mehreren oder allen substituierbaren Positionen des Grundgerüsts substituiert sein. Geeignete Substituenten sind dieselben, die bereits bezüglich der Arylgruppen genannt wurden.

Unter einem Alkylrest oder einer Alkylgruppe ist ein Rest mit 1 bis 20 Kohlenstoffatomen, bevorzugt 1 bis 10 Kohlenstoffatomen, besonders bevorzugt 1 bis 8 Kohlenstoffatomen zu verstehen. Dieser Alkylrest kann verzweigt oder unverzweigt sein und gegebenenfalls mit einem oder mehreren Heteroatomen, bevorzugt N, O oder S unterbrochen sein. Des Weiteren kann dieser Alkylrest mit einem oder mehreren der bezüglich der Arylgruppen genannten Substituenten substituiert sein. Es ist ebenfalls möglich, dass der Alkylrest eine oder mehrere Arylgruppen trägt. Dabei sind alle der vorstehend aufgeführten Arylgruppen geeignet. Besonders bevorzugt sind die Alkylreste ausgewählt aus der Gruppe bestehend aus Methyl, Ethyl, i-Propyl, n-Propyl, i-Butyl, n-Butyl, t-Butyl, sec-Butyl, i-Pentyl, n-Pentyl, sec-Pentyl, neo-Pentyl, n-Hexyl, i-Hexyl und sec-Hexyl. Ganz besonders bevorzugt sind Methyl, i-Propyl, tert-Butyl und n-Hexyl, insbesondere Methyl.

Unter einem Cycloalkylrest oder einer Cycloalkylgruppe ist ein cyclischer Rest mit 3 bis 20 Kohlenstoffatomen, bevorzugt 3 bis 10 Kohlenstoffatomen, besonders bevorzugt 3 bis 8 Kohlenstoffatomen zu verstehen. Dieser Cycloalkylrest kann gegebenenfalls mit einem oder mehreren Heteroatomen, bevorzugt N, O oder S unterbrochen sein. Des Weiteren kann dieser Cycloalkylrest unsubstituiert oder substituiert sein, d. h. mit einem oder mehreren der bezüglich der Arylgruppen genannten Substituenten substituiert sein. Es ist ebenfalls möglich, dass der Cycloalkylrest eine oder mehrere Arylgruppen trägt. Dabei sind alle der vorstehend aufgeführten Arylgruppen geeignet.

Das bezüglich der Aryl-, Heteroaryl, Alkyl- und Cycloalkylreste Gesagte trifft erfindungsgemäß unabhängig voneinander auf die Reste R¹, R², R³, R⁴, R⁵, R⁶, R⁷, R⁸, R⁹ und R¹⁰ zu, wobei R⁵ und R⁶ für den Fall, dass A gleich N ist, ein freies Elektronenpaar bedeuten, d. h., dass an diesen Ring-Stickstoffatomen kein Substituent ausgewählt aus der oben genannten Gruppe vorliegt. Für den Fall, dass A gleich C ist, liegen als R⁵ und R⁶ unabhängig voneinander Wasserstoff und/oder die genannten Substituenten vor.

In einer weiteren Ausführungsform der dinuklearen Pt-Carben-Komplexe der allgemeinen Formeln (Ia) bilden R⁴ und R⁵ oder R⁵ und R⁶ zusammen mit N und A oder mit A und A einen gegebenenfalls von wenigstens einem weiteren Heteroatom unterbrochenen gesättigten, ungesättigten oder aromatischen Ring mit insgesamt 5 bis 18 Kohlenstoff- und/oder Heteroatomen, oder R⁷ und R⁸ oder R⁸ und R⁹ bilden zusammen einen gegebenenfalls von wenigstens einem Heteroatom unterbrochenen gesättigten, ungesättigten oder aromatischen, gegebenenfalls substituierten, Ring mit insgesamt 5 bis 30 Kohlenstoff- und/oder Heteroatomen.

"Insgesamt" bedeutet in diesem Zusammenhang gemäß der vorliegenden Erfindung, dass die Ringatome A mitgezählt werden. Erfindungsgemäß bilden nur die Reste R⁴ und R⁵ oder R⁵ und R⁶ oder R⁷ und R⁸ oder R⁸ und R⁹ entsprechende Ringe, die sich am gleichen Fünfring oder Sechsring befinden.

In einer bevorzugten Ausführungsform bilden R⁷ und R⁸ zusammen einen gegebenenfalls von wenigstens einem Heteroatom unterbrochenen gesättigten, ungesättigten oder aromatischen Ring mit insgesamt 5 bis 18 Kohlenstoff und/oder Heteroatomen. Beispielsweise bilden R⁷ und R⁸ ein Ringsystem der folgenden Formel (IIa) oder (IIb)

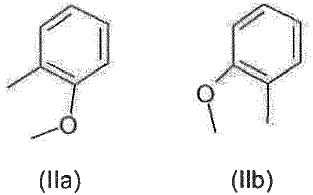

In einer weiteren bevorzugten Ausführungsform bilden R⁵ und R⁶ gemeinsam mit A und A ein Ringsystem der allgemeinen Formeln (IIc) 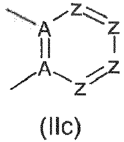 mit Z unabhängig voneinander CR' oder N, mit R' gleich Wasserstoff, linearer oder verzweigter, gegebenenfalls von wenigstens einem Heteroatom unterbrochener, gegebenenfalls wenigstens eine funktionelle Gruppe tragender Alkylrest mit 1 bis 10 Kohlenstoffatomen, Cycloalkylrest mit 3 bis 10 Kohlenstoffatomen, substituierter oder unsubstituierter Arylrest mit 6 bis 18 Kohlenstoffatomen, substituierter oder unsubstituierter Heteroarylrest mit insgesamt 5 bis 18 Kohlenstoff- und/oder Heteroatomen, funktionelle Gruppe ausgewählt aus Ether-, Amino-, Thio-, Ester-, Carbonyl-, Nitro- oder Halogen-Gruppe.

In einer besonders bevorzugten Ausführungsform bilden R⁶ und R⁷ einen Phenylring, entsprechend der Formel (IId)

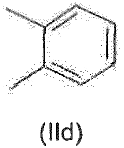

Die dinuklearen Pt-Carben-Komplexe der allgemeinen Formel (Ia) enthalten zwei Atome Platin. In den erfindungsgemäßen dinuklearen Pt-Carben-Komplexen der allgemeinen Formel (Ia) liegen die Platinatome in der Oxidationsstufe +II vor.

In einer besonders bevorzugten Ausführungsform der erfindungsgemäßen Pt-Carben-Komplexe der allgemeinen Formel (Ia) weisen A, R¹, R², R³, R⁴, R⁵, R⁶, R⁷, R⁸, R⁹ und R¹⁰ die folgenden Bedeutungen auf:
- A: unabhängig voneinander N oder C,
- R¹, R³: unabhängig voneinander Wasserstoff, linearer oder verzweigter Alkylrest mit 1 bis 4 Kohlenstoffatomen,
- R²: Wasserstoff
- R⁴: unabhängig voneinander, linearer oder verzweigter Alkylrest mit 1 bis 4 Kohlenstoffatomen, substituierter oder unsubstituierter Arylrest mit 6 Kohlenstoffatomen, substituierter oder unsubstituierter Heteroarylrest mit 5 Kohlenstoff und/oder Heteroatomen,
- R⁵, R⁶: freies Elektronenpaar, falls A gleich N, oder, falls A gleich C, unabhängig voneinander Wasserstoff, linearer oder verzweigter Alkylrest mit 1 bis 4 Kohlenstoffatomen, substituierter oder unsubstituierter Arylrest mit 6 Kohlenstoffatomen, substituierter oder unsubstituierter Heteroarylrest mit 5 Kohlenstoff und/oder Heteroatomen,
oder
R⁵ und R⁶ bilden zusammen mit A und A einen gegebenenfalls von wenigstens einem weiteren Heteroatom unterbrochenen aromatischen Ring mit insgesamt 5 bis 10 Kohlenstoff- und/oder Heteroatomen,
- R⁷, R⁸, R⁹, R¹⁰: unabhängig voneinander Wasserstoff
oder
R⁷und R⁸ bilden zusammen einen gegebenenfalls von wenigstens einem Heteroatom unterbrochenen ungesättigten oder aromatischen, gegebenenfalls substituierten, Ring mit insgesamt 5 bis 12 Kohlenstoff- und/oder Heteroatomen.

In einer ganz besonders bevorzugten Ausführungsform der vorliegenden Erfindung entsprechen die erfindungsgemäßen dinuklearen Pt-Carben-Komplexe den folgenden Formeln (Ia'), (Ib'), (Ic') (Id'), (Ie') oder (If'):

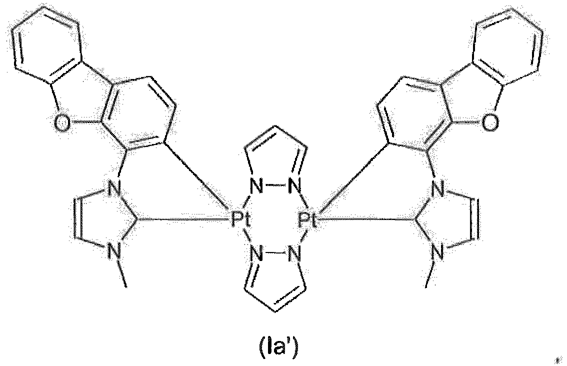

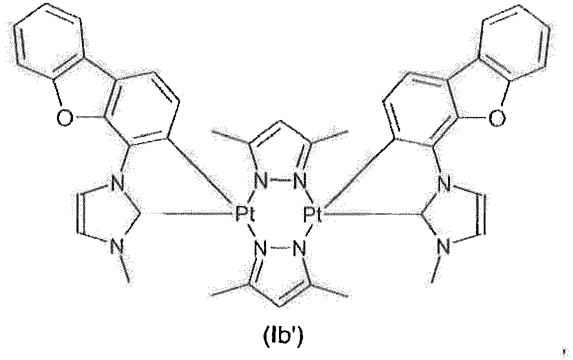

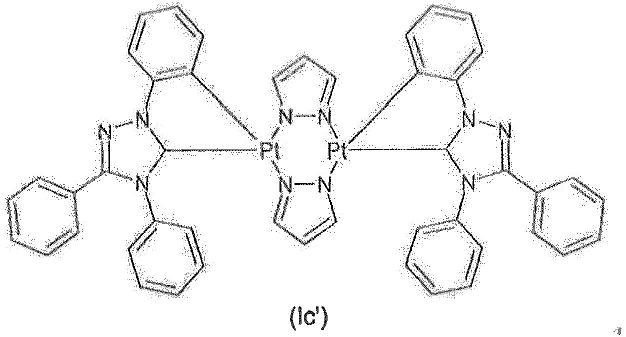

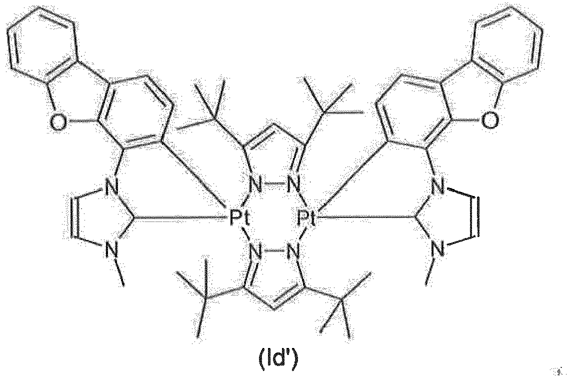

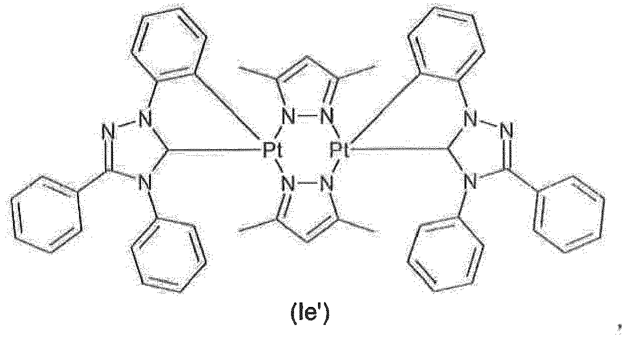

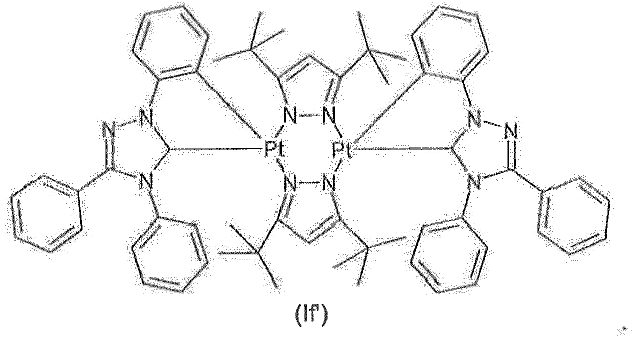

Die vorstehend genannten dinuklearen Pt-Carben-Komplexe und Mischungen davon sind hervorragend als Emittermoleküle in organischen Licht-emittierenden Dioden (OLEDs) geeignet. Durch Variationen der Liganden ist es möglich, entsprechende Komplexe bereitzustellen, die Elektrolumineszenz im roten, grünen sowie insbesondere im blauen Bereich des elektromagnetischen Spektrums zeigen. Die erfindungsgemäß verwendeten neutralen Pt-Carben-Komplexe eignen sich daher für den Einsatz in technisch verwendbaren Vollfarbendisplays oder weißen OLEDs als Beleuchtungsmittel.

Die vorliegende Erfindung betrifft auch ein Verfahren zur Herstellung der erfindungsgemäßen dinuklearen Pt-Carben-Komplexe durch Inkontaktbringen von geeigneten Pt-Verbindungen mit den entsprechenden Liganden bzw. Ligandvorläufern und/oder Pyrazol bzw. entsprechenden Pyrazol-Derivaten.

Im Prinzip kann das erfindungsgemäße Verfahren zur Herstellung der Pt-Carben-Komplexe der allgemeinen Formel (Ia) in zwei Ausführungsformen erfolgen.

In der ersten Ausführungsform (Variante A) des erfindungsgemäßen Verfahrens werden geeignete Pt-Verbindungen, d. h. -Salze bzw. -Komplexe mit geeigneten Ligandvorläufern und Pyrazol bzw. entsprechenden Pyrazol-Derivaten umgesetzt.

Im Allgemeinen sind alle dem Fachmann bekannten Pt-Salze geeignet, die unter den erfindungsgemäßen Reaktionsbedingungen eine genügend hohe Reaktivität zeigen. Bevorzugt sind entsprechende Pt-Salze bzw. -Komplexe ausgewählt aus der Gruppe bestehend aus Pt(COD)Cl₂ (COD = Cyclooctadien), Pt(PPh₃)₂Cl₂, Pt(Pyridin)₂Cl₂, Pt(Phenanthrolin)Cl₂, Pt(NH₃)₂Cl₂, Pt(acac)₂, PtCl₂, K₂PtCl₄ und Mischungen davon, besonders bevorzugt wird Pt(COD)Cl₂ eingesetzt.

Geeignete Ligandvorläufer sind Verbindungen, die, nach Reaktion mit Pt-Verbindungen und Pyrazol bzw. dessen Derivaten die dinuklearen Pt-Carben-Komplexe der allgemeinen Formel (Ia) ergeben. In einer bevorzugten Ausführungsform entsprechend geeignete Ligandvorläufer der allgemeinen Formel (III) 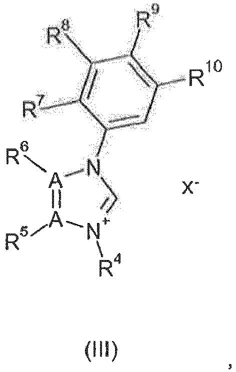 worin R⁴, R⁵, R⁶, R⁷, R⁸, R⁹, R¹⁰ und A die gleichen Bedeutungen haben wie bezüglich Formeln (Ia) oder (Ib) genannt. Dem Fachmann ist klar, dass die Reste R⁴, R⁵, R⁶, R⁷, R⁸, R⁹, R¹⁰ und A so auszuwählen sind, dass die gewünschte Verbindung der allgemeinen Formel (Ia) erhalten wird.

In der Verbindung der allgemeinen Formel (III) bedeutet X⁻ beispielsweise Halogenid, insbesondere Chlorid, Bromid, Iodid, besonders bevorzugt Iodid, oder BF₄⁻, PF₆⁻, SbF₆⁻, ClO₄⁻, ½ SO₄²⁻, bevorzugt BF₄⁻, PF₆⁻.

Besonders bevorzugte Verbindungen der allgemeinen Formel (III), die in dem erfindungsgemäßen Verfahren als Ligandvorläufer eingesetzt werden, sind solche Verbindungen, die die oben genannten bevorzugten Reste R⁴, R⁵, R⁶, R⁷, R⁸, R⁹, R¹⁰ und A enthalten und in denen X⁻ gleich I⁻ ist, ganz besonders bevorzugt die folgenden Verbindungen (lila) und (IIIb): 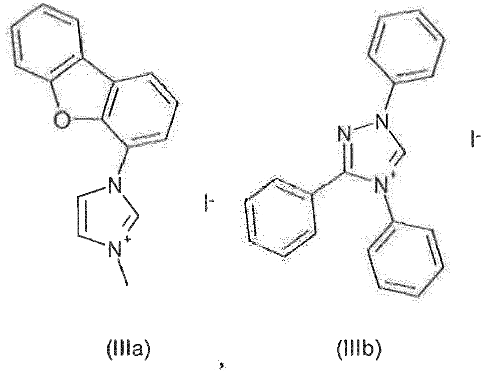 Entsprechende Verbindungen der allgemeinen Formel (IIIa) und (IIIb) sind nach dem Fachmann bekannten Verfahren zugänglich.

Pyrazol bzw. entsprechende Derivate entsprechen erfindungsgemäß der allgemeinen Formel (IV1) bzw. (IV2): 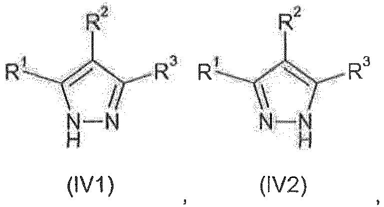 wobei Pyrazol in zwei tautomeren Formen (IV1) und (IV2) auftreten kann. Bei allen Verbindungen der allgemeinen Formel (IV) sollen erfindungsgemäß jeweils beide tautomeren Formen 1 und 2 umfasst sein.

In den allgemeinen Formeln (IV1) und (IV2) weisen R¹, R² und R³ die oben genannten Bedeutungen und bevorzugten Bedeutungen auf. Dem Fachmann ist klar, dass die Reste R¹, R² und R³ so auszuwählen sind, dass die gewünschte Verbindung der allgemeinen Formel (Ia) erhalten wird.

Besonders bevorzugte Verbindungen der allgemeinen Formel (IV), die in dem erfindungsgemäßen Verfahren eingesetzt werden, sind die folgenden Verbindungen (IVa), (IVb), (IVc), (IVd), (IVe) und (IVf). wobei jeweils auch die tautomeren Formen mit umfasst sind: 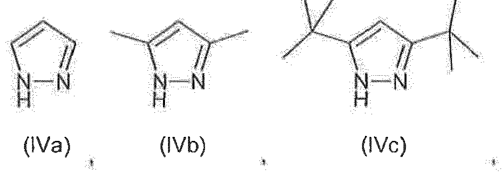 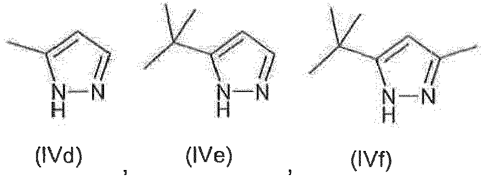 Entsprechende Verbindungen der allgemeinen Formel (IV) sind nach dem Fachmann bekannten Verfahren zugänglich bzw. kommerziell erhältlich.

Das molare Verhältnis der eingesetzten Verbindungen in dieser Ausführungsform des erfindungsgemäßen Verfahrens wird bevorzugt so bemessen, dass entsprechende Verbindungen der allgemeinen Formel (Ia) erhalten werden, beispielsweise 1 bis 10 eq, bevorzugt 1 bis 5 eq, besonders bevorzugt 1 bis 2 eq, Ligand, 1 eq Metall und 1 bis 10 eq, bevorzugt 1 bis 5 eq, besonders bevorzugt 2 bis 4 eq, Pyrazol bzw.-Pyrazolderivat.

Die Umsetzung erfolgt im Allgemeinen bei einer Temperatur von 0 bis 150°C, bevorzugt 0 bis 120 °C, beispielsweise Raumtemperatur, 100°C oder 115 °C.

Bevorzugt wird das erfindungsgemäße Verfahren in einem Lösungsmittel durchgeführt. Geeignete Lösungsmittel sind dem Fachmann bekannt, beispielsweise Ether, cyclische Ether, Ketone, polare Lösungsmittel, bevorzugt Dioxan, Butanon, Ethoxyethanol, Dimethylformamid (DMF) oder Mischungen davon.

Die Reaktionsdauer ist abhängig von dem gewünschten Pt-Carben-Komplex und beträgt im Allgemeinen 1 bis 80 Stunden, bevorzugt 2 bis 70 Stunden, besonders bevorzugt 10 bis 60 Stunden, beispielsweise 54 Stunden.

Der erhaltene Pt-Carben-Komplex der allgemeinen Formel (Ia) kann nach dem Fachmann bekannten Methoden aufgearbeitet werden. Beispielsweise wird das während der Umsetzung ausgefallene Produkt filtriert, gegebenenfalls gewaschen, z. B. mit Wasser und anschließend säulenchromatographisch mit Dichlormethan aufgereinigt und getrocknet.

In einer zweiten Ausführungsform (Variante B) können die erfindungsgemäßen dinuklearen Pt-Carbenkomplexe erhalten werden, indem Pt-Komplexe, in denen die in den entsprechenden Verbindungen der allgemeinen Formel (Ia) enthaltenen Liganden vorliegen, mit Pyrazol bzw. entsprechenden Derivaten davon umgesetzt werden.

Pt-Komplexe, in denen die in den entsprechenden gewünschten Verbindungen der allgemeinen Formel (Ia) enthaltenen Liganden vorliegen, entsprechen in einer bevorzugten Ausführungsform der folgenden allgemeinen Formel (V): 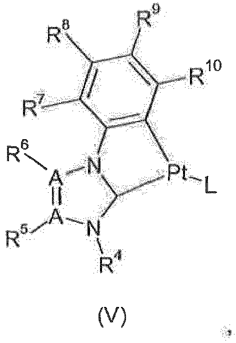 in der R⁴, R⁵, R⁶, R⁷, R⁸, R⁹, R¹⁰ und A die oben genannten Bedeutungen und bevorzugten Bedeutungen aufweisen. Dem Fachmann ist klar, dass die Reste R⁴, R⁵, R⁶, R⁷, R⁸, R⁹, R¹⁰ und A so auszuwählen sind, dass die gewünschte Verbindung der allgemeinen Formel (Ia) erhalten wird.

In der allgemeinen Formel (V) bedeutet L einen oder mehrere, ein- oder mehrzähnige Liganden, die in der Verbindung der allgemeinen Formel (V) an das Pt gebunden sind. Geeignete Liganden, die während der Umsetzung mit Pyrazol bzw. dem Pyrazolderivat unter den erfindungsgemäßen Reaktionsbedingungen leicht abgespalten werden, sind dem Fachmann bekannt, beispielsweise cyclooctadien (cod), Cl, Br, Acetylacetonat (acac), 1,3-Diketiminate (nacnac), OAc (Acetat), BF₄, PF₆, PPh₃ oder Lösungsmittel.

Besonders bevorzugt bedeutet L einen zweizähnigen Liganden der folgenden Formel (VI): 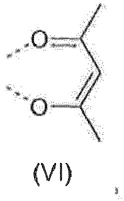 wobei Bindungen an das Pt-Atom über die gestrichelten Bindungen erfolgen.

In dieses erfindungsgemäße Verfahren einsetzbares Pyrazol bzw. entsprechende Pyrazolderivate sind die oben genannten Verbindungen der allgemeinen Formel (IV).

Das molare Verhältnis der eingesetzten Verbindungen in dieser Ausführungsform des erfindungsgemäßen Verfahrens wird bevorzugt so bemessen, dass entsprechende Verbindungen der allgemeinen Formel (Ia) erhalten werden, beispielsweise 1 eq Komplex und 1 bis 10 eq, bevorzugt 1 bis 5 eq, besonders bevorzugt 1 bis 2 eq, Pyrazol oder Pyrazolderivat.

Die Umsetzung erfolgt im Allgemeinen bei einer Temperatur von 0 bis 150 °C, bevorzugt 0 bis 120 °C, beispielsweise 60 °C.

Bevorzugt wird das erfindungsgemäße Verfahren in einem Lösungsmittel durchgeführt. Geeignete Lösungsmittel sind dem Fachmann bekannt, beispielsweise Ether, cyclische Ether, Ketone, polare Lösungsmittel, bevorzugt Methylenchlorid, Dioxan, Ethoxyethanol, Butanon, Dimethylformamid (DMF) oder Mischungen davon.

Die Reaktionsdauer ist abhängig von dem gewünschten Pt-Carben-Komplex und beträgt im Allgemeinen 1 bis 50 Stunden, bevorzugt 2 bis 40 Stunden, besonders bevorzugt 10 bis 30 Stunden, beispielsweise 24 Stunden.

Die Aufarbeitung der so erhaltenen dinuklearen Pt-Carben-Komplexe der allgemeinen Formel (Ia) kann wie bereits zu der ersten Ausführungsform beschrieben erfolgen, wobei säulenchromatographische Reinigung mit Dichlormethan bevorzugt ist.

Die erfindungsgemäßen dinuklearen Pt-Carben-Komplexe der allgemeinen Formel (Ia) eignen sich hervorragend als Emittersubstanzen, da sie eine Emission (Elektrolumineszenz) im sichtbaren Bereich des elektromagnetischen Spektrums, beispielsweise bei 440 bis 500 nm, aufweisen. Durch die dinuklearen Pt-Carben-Komplexe ist es möglich, Verbindungen bereitzustellen, die Elektrolumineszenz im roten, grünen sowie im blauen Bereich des elektromagnetischen Spektrums aufweisen.

Somit ist es möglich, mithilfe der erfindungsgemäßen dinuklearen Pt-Carben-Komplexe als Emittersubstanzen technisch einsetzbare Vollfarbendisplays oder weiße OLEDs für Beleuchtungsmittel bereitzustellen.

Des Weiteren weisen die erfindungsgemäßen Verbindungen der allgemeinen Formel (Ia) sehr hohe Quantenausbeuten auf, 60 bis 100%. Die Quantenausbeute wird durch dem Fachmann bekannte Verfahren, beispielsweise durch UV/Vis-Spektroskopie der Emitter in Lösung oder in dünnen Polymerfilmen, bestimmt.

Ein weiterer Gegenstand der vorliegenden Anmeldung ist daher ein OLED, enthaltend wenigstens einen erfindungsgemäßen dinuklearen Pt-Carben-Komplex der allgemeinen Formel (Ia).

Ein weiterer Gegenstand der vorliegenden Anmeldung ist auch die Verwendung der dinuklearen Pt-Carben-Komplexe der allgemeinen Formel (Ia) als Licht-emittierende Schicht in OLEDs, bevorzugt als Emitter, Matrixmaterial, Ladungstransportmaterial und/oder Ladungsblocker.

Organische Licht-emittierende Dioden sind grundsätzlich aus mehreren Schichten aufgebaut:
- Anode (1)
- Löcher-transportierende Schicht (2)
- Licht-emittierende Schicht (3)
- Elektronen-transportierende Schicht (4)
- Kathode (5)

Die dinuklearen Pt-Carben-Komplexe der allgemeinen Formel (Ia) werden bevorzugt in der Licht-emittierenden Schicht (3) als Emittermoleküle eingesetzt.

Ein weiterer Gegenstand der vorliegenden Anmeldung ist daher eine Licht-emittierende Schicht enthaltend mindestens einen der erfindungsgemäßen dinuklearen Pt-Carben-Komplexe der allgemeinen Formel (Ia), bevorzugt als Emittermolekül. Bevorzugte dinukleare Pt-Carben-Komplexe der allgemeinen Formel (Ia) sind bereits vorstehend genannt.

Die erfindungsgemäß verwendeten dinuklearen Pt-Carben-Komplexe der allgemeinen Formel (Ia) können in Substanz, d. h. ohne weitere Zusätze, in der Licht-emittierenden Schicht vorliegen. Es ist jedoch auch möglich, dass neben den erfindungsgemäß eingesetzten dinuklearen Pt-Carben-Komplexen der allgemeinen Formel (Ia) weitere Verbindungen in der Licht-emittierenden Schicht vorliegen. Beispielsweise kann ein fluoreszierender Farbstoff anwesend sein, um die Emissionsfarbe des als Emittermoleküls eingesetzten dinuklearen Pt-Carben-Komplexes zu verändern. Des Weiteren kann ein Verdünnungsmaterial (Matrixmaterial) eingesetzt werden. Dieses Verdünnungsmaterial kann ein Polymer sein, z. B. Poly(N-vinylcarbazol) oder Polysilan. Das Verdünnungsmaterial kann jedoch ebenfalls ein kleines Molekül sein, z. B. 4,4'-N,N'-Dicarbazolbiphenyl (CDP) oder tertiäre aromatische Amine. Wenn ein Verdünnungsmaterial eingesetzt wird, beträgt der Anteil der erfindungsgemäß eingesetzten dinuklearen Pt-Carben-Komplexe in der Licht-emittierenden Schicht im Allgemeinen weniger als 40 Gew.-%, bevorzugt 3 bis 20 Gew.-%. Bevorzugt werden die erfindungsgemäßen dinuklearen Pt-Carben-Komplexe der allgemeinen Formel (Ia) in einem Matrixmaterial eingesetzt. Somit enthält die Licht-emittierende Schicht bevorzugt mindestens einen erfindungsgemäßen dinuklearen Pt-Carben-Komplex der allgemeinen Formel (Ia) und ein Matrixmaterial.

Ein weiterer Gegenstand der vorliegenden Anmeldung ist eine Licht-emittierende Schicht enthaltend mindestens einen dinuklearen Pt-Carben-Komplex der allgemeinen Formel (Ia) als Emittermolekül. Bevorzugte Komplexe der allgemeinen Formel (Ia) wurden bereits vorstehend genannt.

Die einzelnen der vorstehend genannten Schichten des OLEDs können wiederum aus zwei oder mehreren Schichten aufgebaut sein. Beispielsweise kann die Löcher-transportierende Schicht aus einer Schicht aufgebaut sein, in die aus der Elektrode Löcher injiziert werden und einer Schicht, die die Löcher von der Loch-injizierenden Schicht weg in die Licht-emittierende Schicht transportiert. Die Elektronen-transportierende Schicht kann ebenfalls aus mehreren Schichten bestehen, z. B. einer Schicht, worin Elektronen durch die Elektrode injiziert werden, und einer Schicht, die aus der Elektronen-injizierenden Schicht Elektronen erhält und in die Licht-emittierende Schicht transportiert. Diese genannten Schichten werden jeweils nach Faktoren wie Energieniveau, Temperaturresistenz und Ladungsträgerbeweglichkeit sowie Energiedifferenz der genannten Schichten mit den organischen Schichten oder den Metallelektroden ausgewählt. Der Fachmann ist in der Lage, den Aufbau der OLEDs so zu wählen, dass er optimal an die erfindungsgemäß als Emittersubstanzen verwendeten dinuklearen Pt-Carben-Komplexe gemäß der vorliegenden Erfindung angepasst ist.

Um besonders effiziente OLEDs zu erhalten, sollte das HOMO (höchstes besetztes Molekülorbital) der Loch-transportierenden Schicht mit der Arbeitsfunktion der Anode angeglichen sein und das LUMO (niedrigstes unbesetztes Molekülorbital) der elektronentransportierenden Schicht sollte mit der Arbeitsfunktion der Kathode angeglichen sein.

Ein weiterer Gegenstand der vorliegenden Anmeldung ist ein OLED enthaltend mindestens eine erfindungsgemäße Licht-emittierende Schicht. Die weiteren Schichten in dem OLED können aus einem beliebigen Material aufgebaut sein, das üblicherweise in solchen Schichten eingesetzt wird und dem Fachmann bekannt ist.

Die Anode ist eine Elektrode, die positive Ladungsträger bereitstellt. Sie kann zum Beispiel aus Materialien aufgebaut sein, die ein Metall, eine Mischung verschiedener Metalle, eine Metalllegierung, ein Metalloxid oder eine Mischung verschiedener Metalloxide enthält. Alternativ kann die Anode ein leitendes Polymer sein. Geeignete Metalle umfassen die Metalle der Gruppen 11, 4, 5 und 6 des Periodensystems der Elemente sowie die Übergangsmetalle der Gruppen 8 bis 10. Wenn die Anode lichtdurchlässig sein soll, werden im Allgemeinen gemischte Metalloxide der Gruppen 12, 13 und 14 des Periodensystems der Elemente eingesetzt, zum Beispiel Indium-Zinn-Oxid (ITO). Es ist ebenfalls möglich, dass die Anode (1) ein organisches Material, zum Beispiel Polyanilin enthält, wie beispielsweise in Nature, Vol. 357, Seiten 477 bis 479 (11. Juni 1992) beschrieben ist. Zumindest entweder die Anode oder die Kathode sollten mindestens teilweise transparent sein, um das gebildete Licht auskoppeln zu können.

Geeignete Lochtransportmaterialien für die Schicht (2) des erfindungsgemäßen OLEDs sind zum Beispiel in Kirk-Othmer Encyclopedia of Chemical Technology, 4. Auflage, Vol. 18, Seiten 837 bis 860, 1996 offenbart. Sowohl Löcher transportierende Moleküle als auch Polymere können als Lochtransportmaterial eingesetzt werden. Üblicherweise eingesetzte Löcher transportierende Moleküle sind ausgewählt aus der Gruppe bestehend aus 4,4'-Bis[N-(1-naphthyl)-N-phenyl-amino]biphenyl (NPD), N, N'-Diphenyl-N,N'-Bis(3-methylphenyl)-[1,1'-biphenyl]-4,4'-diamin (TPD), 1,1-Bis[(di-4-tolylamino)-phenyl]cyclohexan (TAPC), N,N'-Bis(4-methylphenyl)-N,N'-Bis(4-ethylphenyl)-[1,1'-(3,3'-dimethyl)biphenyl]-4,4'-diamin (ETPD), Tetrakis-(3-methylphenyl)-N,N,N',N'-2,5-phenylendiamin (PDA), α-Phenyl-4-N,N-diphenylaminostyrol (TPS), p-(Diethylamino)-benzaldehyddiphenylhydrazon (DEH), Triphenylamin (TPA), Bis[4-(N,N-diethylamino)-2-methylphenyl)(4-methyl-phenyl)methan (MPMP), 1-Phenyl-3-[p-(diethylamino)styryl]-5-[p-(diethylamino)phenyl]pyrazolin (PPR oder DEASP), 1,2-trans-Bis(9H-carbazol-9-yl)cyclobutan (DCZB), N,N,N',N'-tetrakis(4-methylphenyl)-(1,1'-biphenyl)-4,4'-diamin (TTB) und Porphyrinverbindungen wie Kupferphthalocyanine. Üblicherweise eingesetzte Löcher transportierende Polymere sind ausgewählt aus der Gruppe bestehend aus Polyvinylcarbazolen, (Phenylmethyl)polysilanen und Polyanilinen. Es ist ebenfalls möglich, Löcher transportierende Polymere durch Dotieren Löcher transportierender Moleküle in Polymere wie Polystyrol und Polycarbonat zu erhalten. Geeignete Löcher transportierende Moleküle sind die bereits vorstehend genannten Moleküle.

Geeignete Elektronen transportierende Materialien für die Schicht (4) der erfindungsgemäßen OLEDs umfassen mit oxinoiden Verbindungen chelatisierte Metalle wie Tris(8-hydroxychinolato)aluminium (Alq3), Verbindungen auf Phenanthrolinbasis wie 2,9-Dimethyl, 4,7-Diphenyl-1, 10-phenanthrolin (DDPA) oder 4,7-Diphenyl-1,10-phenanthrolin (DPA) und Azolverbindungen wie 2-(4-Biphenylyl)-5-(4-t-butylphenyl)-1,3,4-oxadiazol (PBD) und 3-(4-Biphenylyl)-4-phenyl-5-(4-t-butylphenyl)-1,2,4-triazol (TAZ). Dabei kann die Schicht (4) sowohl zur Erleichterung des Elektronentransports dienen als auch als Pufferschicht oder als Sperrschicht, um ein Quenchen des Excitons an den Grenzflächen der Schichten des OLEDs zu vermeiden. Vorzugsweise verbessert die Schicht (4) die Beweglichkeit der Elektronen und reduziert ein Quenchen des Excitons.

Die Kathode (5) ist eine Elektrode, die zur Einführung von Elektronen oder negativen Ladungsträgern dient. Die Kathode kann jedes Metall oder Nichtmetall sein, das eine geringere Arbeitsfunktion aufweist als die Anode. Geeignete Materialien für die Kathode sind ausgewählt aus der Gruppe bestehend aus Alkalimetallen der Gruppe 1, zum Beispiel Li, Cs, Erdalkalimetallen der Gruppe 2, Metallen der Gruppe 12 des Periodensystems der Elemente, umfassend die Seltenerdmetalle und die Lanthanide und Aktinide. Des Weiteren können Metalle wie Aluminium, Indium, Calcium, Barium, Samarium und Magnesium sowie Kombinationen davon eingesetzt werden. Weiterhin können Lithium enthaltende organometallische Verbindungen oder LiF zwischen der organischen Schicht und der Kathode aufgebracht werden, um die Betriebsspannung (Operating Voltage) zu vermindern.

Das OLED gemäß der vorliegenden Erfindung kann zusätzlich weitere Schichten enthalten, die dem Fachmann bekannt sind. Beispielsweise kann zwischen der Schicht (2) und der Licht emittierenden Schicht (3) eine Schicht aufgebracht sein, die den Transport der positiven Ladung erleichtert und/oder die Bänderlücke der Schichten aneinander anpasst. Alternativ kann diese weitere Schicht als Schutzschicht dienen. In analoger Weise können zusätzliche Schichten zwischen der Licht emittierenden Schicht (3) und der Schicht (4) vorhanden sein, um den Transport der negativen Ladung zu erleichtern und/oder die Bänderlücke zwischen den Schichten aneinander anzupassen. Alternativ kann diese Schicht als Schutzschicht dienen.

In einer bevorzugten Ausführungsform enthält das erfindungsgemäße OLED zusätzlich zu den Schichten (1) bis (5) mindestens eine der im Folgenden genannten weiteren Schichten:
- eine Loch-Injektionsschicht zwischen der Anode (1) und der Löcher-transportierenden Schicht (2);
- eine Blockschicht für Elektronen zwischen der Löcher-transportierenden Schicht (2) und der Licht-emittierenden Schicht (3);
- eine Blockschicht für Löcher zwischen der Licht-emittierenden Schicht (3) und der Elektronen-transportierenden Schicht (4);
- eine Elektronen-Injektionsschicht zwischen der Elektronen-transportierenden Schicht (4) und der Kathode (5).

Dem Fachmann ist bekannt, wie er (zum Beispiel auf Basis von elektrochemischen Untersuchungen) geeignete Materialien auswählen muss. Geeignete Materialien für die einzelnen Schichten sind dem Fachmann bekannt und z. B. in WO 00/70655 offenbart.

Des Weiteren ist es möglich, dass einige oder alle der Schichten (1), (2), (3), (4) und (5) oberflächenbehandelt sind, um die Effizienz des Ladungsträgertransports zu erhöhen. Die Auswahl der Materialien für jede der genannten Schichten ist bevorzugt dadurch bestimmt, ein OLED mit einer hohen Effizienz zu erhalten.

Die Herstellung des erfindungsgemäßen OLEDs kann nach dem Fachmann bekannten Methoden erfolgen. Im Allgemeinen wird das OLED durch aufeinanderfolgende Dampfabscheidung (Vapor deposition) der einzelnen Schichten auf ein geeignetes Substrat hergestellt. Geeignete Substrate sind zum Beispiel Glas oder Polymerfilme. Zur Dampfabscheidung können übliche Techniken eingesetzt werden wie thermische Verdampfung, Chemical Vapor Deposition und andere. In einem alternativen Verfahren können die organischen Schichten aus Lösungen oder Dispersionen in geeigneten Lösungsmitteln beschichtet werden, wobei dem Fachmann bekannte Beschichtungstechniken angewendet werden.

Im Allgemeinen haben die verschiedenen Schichten folgende Dicken: Anode (2) 500 bis 5000 Å, bevorzugt 1000 bis 2000 Å; Löcher-transportierende Schicht (3) 50 bis 1000 A, bevorzugt 200 bis 800 Å, Licht-emittierende Schicht (4) 10 bis 1000 A, bevorzugt 100 bis 800 Å, Elektronen transportierende Schicht (5) 50 bis 1000 A, bevorzugt 200 bis 800 Å, Kathode (6) 200 bis 10.000 A, bevorzugt 300 bis 5000 A. Die Lage der Rekombinationszone von Löchern und Elektronen in dem erfindungsgemäßen OLED und somit das Emissionsspektrum des OLED können durch die relative Dicke jeder Schicht beeinflusst werden. Das bedeutet, die Dicke der Elektronentransportschicht sollte bevorzugt so gewählt werden, dass die Elektronen/Löcher Rekombinationszone in der Licht-emittierenden Schicht liegt. Das Verhältnis der Schichtdicken der einzelnen Schichten in dem OLED ist von den eingesetzten Materialien abhängig. Die Schichtdicken von gegebenenfalls eingesetzten zusätzlichen Schichten sind dem Fachmann bekannt.

Die erfindungsgemäßen OLEDs können in allen Vorrichtungen eingesetzt werden, worin Elektrolumineszenz nützlich ist. Geeignete Vorrichtungen sind bevorzugt ausgewählt aus stationären und mobilen Bildschirmen. Die vorliegende Erfindung betrifft daher auch eine Vorrichtung ausgewählt aus der Gruppe bestehend aus stationären Bildschirmen und mobilen Bildschirmen, enthaltend ein erfindungsgemäßes OLED.

Stationäre Bildschirme sind z. B. Bildschirme von Computern, Fernsehern, Bildschirme in Druckern, Küchengeräten sowie Reklametafeln, Beleuchtungen und Hinweistafeln. Mobile Bildschirme sind z.B. Bildschirme in Handys, Laptops, Fahrzeugen sowie Zielanzeigen an Bussen und Bahnen.

Weiterhin können die erfindungsgemäßen dinuklearen Pt-Carben-Komplexe der allgemeinen Formel (Ia) in OLEDs mit inverser Struktur eingesetzt werden. Bevorzugt werden die erfindungsgemäßen Komplexe in diesen inversen OLEDs wiederum in der Licht-emittierenden Schicht, besonders bevorzugt als Licht-emittierende Schicht ohne weitere Zusätze, eingesetzt. Der Aufbau von inversen OLEDs und die üblicherweise darin eingesetzten Materialien sind dem Fachmann bekannt.

Die vorliegende Erfindung betrifft auch ein organisches Elektronikbauteil enthaltend wenigstens einen erfindungsgemäßen dinuklearen Pt-Carben-Komplex der allgemeinen Formel (Ia). Besonders bevorzugt betrifft die vorliegende Erfindung ein entsprechendes organisches Elektronikbauteil, wobei es eine Organische Licht-Emittierende Diode (OLED), Organische Photovoltaik-Zelle (OPV), Organischer Feldeffekttransistor (OFET) oder Licht-Emittierende Elektrochemische Zelle (LEEC) ist.

### Beispiele

Die folgenden Verbindungen gemäß der vorliegenden Erfindung werden gemäß der unten stehenden allgemeinen Vorschrift synthetisiert und photophysikalisch charakterisiert.

Die nachstehend genannten dinuklearen Pt-Carben-Komplexe können als als einzelne Isomere (Einzelverbindungen) oder als Mischungen von zwei oder mehr der möglichen Isomeren vorliegen. Im Folgenden ist jeweils ein Isomer dargestellt, was nicht ausschließen soll, dass die betreffende Verbindung in Form von mehr als einem Isomer vorliegt.

Die Photolumineszenz der emittierenden Komplexe wird in dünnen PMMA-Filmen (Polymethylmethacrylat) mit einer Emitterdotierung von 2% durchgeführt. Die Filme werden wie folgt hergestellt: 2mg/l Emitter werden in einer 10%igen PMMA-Lösung in DCM (Mw 120kD) gelöst und auf einen Objektträger mit einem 60 µm Rakel aufgerakelt. Die Anregung erfolgt bei einer Wellenlänge von 325 nm (HeCd-Laser) senkrecht zum Objektträger und die Detektion der Emission in einem Winkel von 45° mittels Faseroptik im Diodenarrayspektrometer.

### Allgemeine Versuchsdurchführung:

### Variante A:

0,8 mmol des entsprechenden Imidazoliumsalzes (entsprechend Formel III) und 0,4 mmol Silber(I)oxid (0,093 g) werden in 20 mL trockenem Dioxan 16 Stunden bei Raumtemperatur unter Argon gerührt. Nach Zugabe von 10 mL Butanon und 0,8 mmol Cyclooctadienplatindichlorid (0,299 g) wird das Reaktionsgemisch 16 Stunden unter Rückfluss gerührt. Die Mischung wird zur Trockene eingeengt, in 20 mL Dimethylformamid aufgenommen und 3,2 mmol Pyrazol bzw. Pyrazolderivat, sowie 3,2 mmol Kalium-tert-butylat (0,359 g) zugesetzt. Das Reaktionsgemisch wird 16 Stunden bei Raumtemperatur und 6 Stunden bei 100 °C gerührt. Das Lösungsmittel wird entfernt und der Rückstand mit Wasser gewaschen. Anschließend wird das Rohprodukt säulenchromatographisch gereinigt (Kieselgel G60, Dichlormethan).

### Variante B:

0,26 mmol des cyclometallierten Platin(II)acetylacetonat-Komplexes (entsprechend Formel V) werden mit 0,26 mmol Pyrazol bzw. Pyrazolderivat und 0,26 mmol Natriummethanolat (0,014 g) in 10 mL Dichlormethan 24 Stunden unter Argon refluxiert. Nachdem das Lösungsmittel entfernt worden ist, wird das Rohprodukt säulenchromatographisch gereinigt (Kieselgel G60, Dichlormethan).

### Di-[1-(Dibenzofuranyl)-3-methylimidazol-2-yliden-C2,C2']platin(II)di-pyrazolat (Verbindung Ia'))

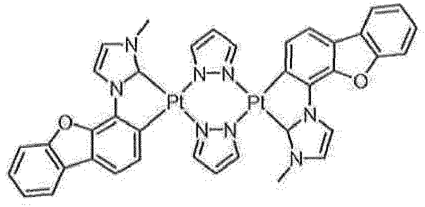

0,8 mmol 1-(Dibenzofuranyl)-3-methylimidazoliumiodid (0,301 g) und 3,2 mmol Pyrazol (0,218 g) reagieren nach Variante A.

| | |
|---|---|
| Summenformel: | C₃₈H₂₈N₈O₂Pt₂ |
| Molare Masse: | 1018,840 g/mol |
| Ausbeute: | 0,168 g (41,2 % d. Th.) |
| Schmelzpunkt: | Zersetzung bei > 357°C |

¹H-NMR (ppm, d₆-DMSO, 300,13 MHz):
δ = 8,11-7,95 (m, 6H, CH); 7,72-7,60 (m, 6H, CH); 7,48-7,45 (m, 4H, CH); 7,39-7,31 (m, 6H, CH); 3,30 (s, 6H, NCH₃)
¹³C-NMR (ppm, CDCl₃, 75,475 MHz):
δ = 154,98 (Cipso); 141,98 (Cipso); 139,25 (CH); 137,89 (CH); 132,61 (Cipso); 130,69 (Cipso); 128,43 (CH); 126,82 (CH); 124,04 (Cipso); 123,24 (CH); 123,10 (CH); 121,42 (Cispo); 120,41 (CH); 117,40 (CH); 116,26 (CH); 111,62 (CH); 105,40 (CH); 35,67 (NCH₃)

### Elementaranalyse für C₃₈H₂₈N₈O₂Pt₂:

| | | | |
|---|---|---|---|
| | C | H | N |
| berechnet | 44,79 % | 2,77 | % 11,00 % |
| gefunden | 44,49 % | 2,57 % | 10,53 % |

oder:
0,26 mmol [1-(Dibenzofuranyl)-3-methylimidazol-2-yliden-C2,C2']platin(II)acetylacetonat (0,142 g) reagieren mit 0,26 mmol Pyrazol (0,018 g) nach Variante B.

| | |
|---|---|
| Summenformel: | C₃₈H₂₈N₈O₂Pt₂ |
| Molare Masse: | 1018,840 g/mol |
| Ausbeute: | 0,118 g (89,1 % d. Th.) |

QY = 73%
λem = 471 nm, 505 nm

### Di-[1,3,5-Triphenyl-1,3,4-triazol-2-yliden-C2,C2']platin(II)di-pyrazolat (Verbindung Ic'))

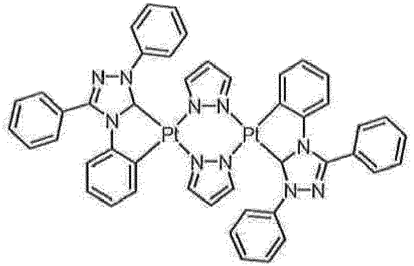

0,8 mmol 1,3,5-Triphenyl-1,3,4-triazoliumiodid (0,340 g) und 3,2 mmol Pyrazol (0,218 g) reagieren nach Variante A.

| | |
|---|---|
| Summenformel: | C₄₆H₃₄N₁₀Pt₂ |
| Molare Masse: | 1116,988 g/mol |
| Ausbeute: | 0,063 g (14,1 % d. Th.) |
| Schmelzpunkt: | Zersetzung bei > 320°C |

¹H-NMR (ppm, d₆-DMSO, 300,13 MHz):
δ = 8,02 (d, 2H, J = 7,6 Hz, CH); 7,59 - 7,27 (m, 32H, CH)
¹³C-NMR (ppm, d₆-DMSO, 75,475 MHz):
δ = 151,20 (Cipso); 145,40 (Cipso); 137,58 (Cipso); 133,20 (Cipso); 130,40 (CH); 129,41 (CH); 129,17 (CH); 129,07 (CH); 128,58 (CH); 128,05 (CH); 128,02 (CH); 126,17 (Cipso); 125,41 (CH); 118,30 (CH)
QY = 63,7%
λem = 444 nm, 473 nm

### Di-[(4-Bromphenyl)-3-methylimidazol-2-yliden-C2,C2']platin(II)di-pyrazolat

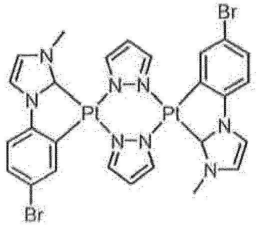

0,8 mmol (4-Bromphenyl)-3-methylimidazoliumiodid (0,292 g) und 3,2 mmol Pyrazol (0,218 g) reagieren nach Variante A.

| | |
|---|---|
| Summenformel: | C₂₆H₂₂N₈Pt₂Br₂ |
| Molare Masse: | 996,480 g/mol |
| Ausbeute: | 0,181 g (45,4 % d. Th.) |
| Schmelzpunkt: | Zersetzung bei > 340 °C |

¹H-NMR (ppm, d₆-DMSO, 300,13 MHz):
δ = 7,91 (d, 2H, J = 2,1 Hz, CH); 7,75 (d, 2H, J = 2,0 Hz, CH); 7,62 (d, 2H, J = 1,9 Hz, CH); 7,33 - 7,25 (m, 4H, CH); 7,20 (d, 2H, J = 2,0 Hz, CH); 7,11 (d, 2H, J = 2,1 Hz, CH); 6,38 (t, 2H, J = 2,0 Hz, CH); 3,21 (s, 6H, NCH₃)
¹³C-NMR (ppm, d₆-DMSO, 75,475 MHz):
δ = 156,23 (Cipso); 146,90 (Cipso); 139,30 (CH); 137,44 (CH); 135,69 (CH); 135,45 (Cipso); 125,61 (CH); 122,93 (CH); 116,88 (Cipso); 115,73 (CH); 112,95 (CH); 105,69 (CH); 35,38 (CH₃)
QY = 8 %
λem = 429 nm, 453 nm

### Di-[(4-Methoxyphenyl)-3-methylimidazol-2-yliden-C2,C2']platin(II)di-pyrazolat

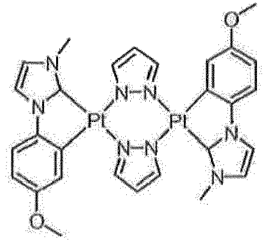

0,8 mmol (4-Methoxyphenyl)-3-methylimidazoliumiodid (0,253 g) und 3,2 mmol Pyrazol (0,218 g) reagieren nach Variante A.

| | |
|---|---|
| Summenformel: | C₂₈H₂₈N₈Pt₂O₂ |
| Molare Masse: | 898,740 g/mol |
| Ausbeute: | 0,040 g (11,1 % d. Th.) |
| Schmelzpunkt: | Zersetzung bei > 320 °C |

¹H-NMR (ppm, d₆-DMSO, 300,13 MHz):
δ = 7,83 (d, 2H, J = 1,8 Hz, CH); 7,80 (d, 2H, J = 1,8 Hz, CH); 7,75 (d, 2H, J = 1,8 Hz, CH); 7,70 (s, 2H, CH); 7,62 (d, 2H, J = 1,5 Hz, CH); 7,21 - 7,16 (m, 4H, CH); 6,53 (d, 2H, J = 8,4 Hz, CH); 6,34 (s, 2H, CH); 3,70 (s, 6H, OCH₃); 3,16 (s, 6H, NCH₃)
¹³C-NMR (ppm, d6-DMSO, 125 MHz):
δ = 167,06 (Cipso-O); 155,88 (Cipso); 141,47 (Cipso); 139,12 (CH); 137,57 (CH); 133,64 (Cipso); 122,33 (CH); 120,23 (CH); 115,28 (CH); 111,33 (CH); 106,64 (CH); 105,30 (CH); 54,75 (OCH₃); 35,44 (NCH₃)
QY = 36 %
λem = 441 nm, 466 nm

### Di-[(4-Methylphenyl)-3-methylimidazol-2-yliden-C2,C2']platin(II)di-pyrazolat

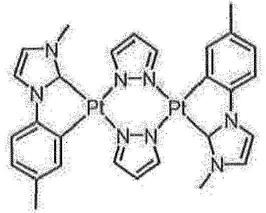

0,8 mmol (4-Methylphenyl)-3-methylimidazoliumiodid (0,240 g) und 3,2 mmol Pyrazol (0,218 g) reagieren nach Variante A.

| | |
|---|---|
| Summenformel: | C₂₈H₂₈N₈Pt₂ |
| Molare Masse: | 866,740 g/mol |
| Ausbeute: | 0,018 g (5,2 % d. Th.) |
| Schmelzpunkt: | Zersetzung bei > 300°C |

¹H-NMR (ppm, d₆-DMSO, 300,13 MHz):
δ = 7,82 (d, 2H, J = 1,9 Hz, CH); 7,69 (d, 2H, J = 1,8 Hz, CH); 7,60 (d, 2H, J = 1,7 Hz, CH); 7,19 (d, 2H, J = 1,7 Hz, CH); 7,13 (d, 2H, J = 7,7 Hz, CH); 6,89 (s, 2H, CH); 6,77 (d, 2H, J = 7,6 Hz, CH); 6,34 (s, 2H, CH); 3,17 (s, 6H, NCH₃); 2,15 (s, 6H, CH₃)
¹³C-NMR (ppm, d₆-DMSO, 75,475 MHz):
δ = 156,71 (Cipso); 145,43 (Cipso); 138,88 (CH); 134,67 (CH); 132,48 (Cipso); 131,72 (Cipso); 123,27 (CH); 122,28 (CH); 115,23 (CH); 110,49 (CH); 105,20 (CH); 35,29 (NCH₃); 21,14 (CH₃)
QY = 31 %
λem = 428 nm, 453 nm

### Di-[(4-Nitrophenyl)-3-methylimidazol-2-yliden-C2,C2']platin(II)di-pyrazolat

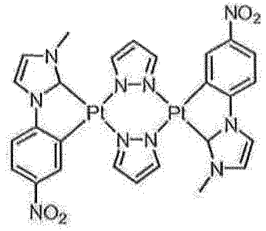

0,8 mmol (4-Nitrophenyl)-3-methylimidazoliumiodid (0,265 g) und 3,2 mmol Pyrazol (0,218 g) reagieren nach Variante A.

| | |
|---|---|
| Summenformel: | C₂₆H₂₂N₁₀O₄Pt₂ |
| Molare Masse: | 928,692 g/mol |
| Ausbeute: | 0,060 g (16,2 % d. Th.) |
| Schmelzpunkt: | Zersetzung bei > 340°C |

¹H-NMR (ppm, d₆-DMSO, 300,13 MHz):
δ = 8,13 (d, 2H, J = 9,1 Hz, CH); 8,03 (d, 2H, J = 1,5 Hz, CH); 8,94 (dd, 2H, J = 8,5 Hz, CH); 7,82 (s, 2H, CH); 7,71 (s, 2H, CH); 7,53 (d, 2H, J = 8,5 Hz, CH); 7,30 (d, 2H, J = 1,3 Hz, CH); 6,45 (s, 2H, CH); 3,24 (s, 6H, NCH₃)
¹³C-NMR (ppm, d6-DMSO, 125 MHz):
δ = 157,58 (Cipso); 153,38 (Cipso); 143,70 (Cipso); 142,82 (CH); 133,87 (Cipso);
128,64 (CH); 127,55 (CH); 125,42 (CH); 120,48 (CH); 118,45 (CH); 109,39 (CH); 106,00 (CH); 35,54 (NCH₃)

### Di-[1-(Dibenzofuranyl)-3-methylimidazol-2-yliden-C2,C2']platin(II)di-3,5-dimethylpyrazolat (Verbindung (Ib'))

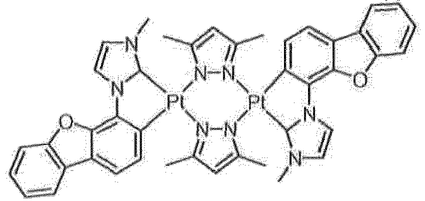

0,8 mmol 1-(Dibenzofuranyl)-3-methylimidazoliumiodid (0,301 g) und 3,2 mmol 3,5-Dimethylpyrazol (0,308 g) reagieren nach Variante A.

| | |
|---|---|
| Summenformel: | C₄₂H₃₆N₈O₂Pt₂ |
| Molare Masse: | 1074,944 g/mol |
| Ausbeute: | 0,026 g (6,1 % d. Th.) |
| Schmelzpunkt: | 221,6 °C |

¹H-NMR (ppm, d₆-DMSO, 300,13 MHz):
δ = 8,03 (d, 2H, J = 2,0 Hz; CH); 7,99 (d, 2H, J = 7,2 Hz, CH); 7,66 (d, 2H, J = 8,1 Hz, CH); 7,59 (d, 2H, J = 7,5 Hz, CH); 7,47 (t, 2H, J = 7,0 Hz, CH); 7,35 (t, 2H, J = 7,6 Hz, CH); 7,34 (d, 2H, J = 2,0 Hz, CH); 7,04 (d, 2H, J = 7,8 Hz, CH); 6,01 (s, 2H, CH); 3,40 (s, 6H, NCH₃); 2,25 (s, 6H, CH₃); 2,20 (s, 6H, CH₃)
¹³C-NMR (ppm, d₆-DMSO, 125 MHz):
δ = 155,06 (Cipso); 145,45 (Cipso); 142,09 (Cipso); 130,84 (Cipso); 129,31 (CH); 126,78 (CH); 124,14 (Cipso); 123,28 (CH); 122,92 (CH); 121,15 (Cispo); 120,40 (CH); 117,63 (CH); 116,26 (CH); 111,67 (CH); 104,20 (CH); 34,83 (NCH3); 14,00 (CH₃); 13,80 (CH₃)
QY = 85 %
λem = 472 nm, 506 nm

### Di-[1-(Dibenzofuranyl)-3-methylimidazol-2-yliden-C2,C2']platin(II)di-3,5-dimethylpyrazolat (Verbindung Id'))

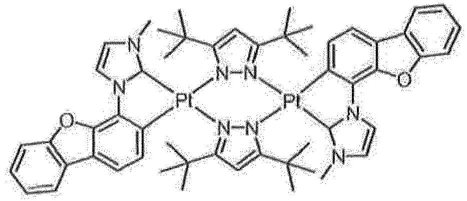

0,8 mmol 1-(Dibenzofuranyl)-3-methylimidazoliumiodid (0,301 g) und 3,2 mmol 3,5-Ditert-butylpyrazol (0,577 g) reagieren nach Variante A.

| | |
|---|---|
| Summenformel: | C₅₄H₆₀N₈O₂Pt₂ |
| Molare Masse: | 1243,256 g/mol |
| Ausbeute: | 0,115 g (23,1 % d. Th.) |
| Schmelzpunkt: | 244°C |

¹H-NMR (ppm, d₆-DMSO, 300,13 MHz):
δ = 7,96 (d, 2H, J = 7,4 Hz; CH); 7,89 (d, 2H, J = 2,0 Hz, CH); 7,61 - 7,52 (m, 4H, CH); 7,45 - 7,32 (m, 6H, CH); 7,20-7,18 (m, 2H, CH); 6,29 (s, 2H, CH); 3,33 (s, 6H, NCH3); 1,44 (s, 18H, CH₃); 1,41 (s, 18H, CH₃)
¹³C-NMR (ppm, d₆-DMSO, 125 MHz):
δ = 158,67 (Cipso); 154,86 (Cipso); 141,51 (Cipso); 133,42 (Cipso); 130,95 (CH); 130,00 (Cipso); 126,52 (CH); 124,08 (Cipso); 123,09 (CH); 122,34 (CH); 120,57 (Cispo); 120,22 (CH); 117,09 (CH); 115,05 (CH); 111,47 (CH); 101,10 (CH); 35,91 (NCH₃); 32,37 (CH₃); 31,29 (CH₃)
QY = 50 %
λem = 512 nm

### Herstellung von OLEDs

Das als Anode verwendete ITO-Substrat wird zuerst mit kommerziellen Reinigungsmitteln für die LCD-Produktion (Deconex® 20NS und Neutralisationsmittel 25ORGAN-ACID®) und anschließend in einem Aceton/Isopropanol-Gemisch im Ultraschallbad gesäubert. Zur Beseitigung möglicher organischer Rückstände wird das Substrat in einem Ozonofen weitere 25 Minuten einem kontinuierlichen Ozonfluss ausgesetzt. Diese Behandlung verbessert auch die Lochinjektionseigenschaften des ITOs. Als nächstes wird die Lochinjektionsschicht AJ20-1000 der Firma Plexcore aus Lösung aufgesponnen.

### Diodenbeispiel 1:

Nach der Lochinjektionsschicht werden die nachfolgend genannten organischen Materialien mit einer Rate von ca. 0.5-5 nm/min bei etwa 10⁻⁷-10⁻⁹ mbar auf das gereinigte Substrat aufgedampft. Als Lochleiter und Excitonenblocker wird Ir(DPBIC)₃ mit einer Dicke von 20 nm auf das Substrat aufgebracht, wovon die ersten 10 nm zur Verbesserung der Leitfähigkeit mit 10% MoOₓ dotiert sind. 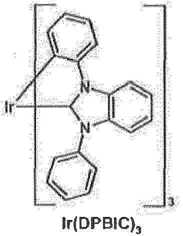 (zur Herstellung von Ir(DPBIC)₃ siehe Ir-Komplex (7) in der Anmeldung PCT/EP/04/ 09269).

Anschließend wird eine Mischung aus 20% Di-[1,3,5-Triphenyl-1,3,4-triazol-2-yliden-C2,C2']platin(II)di-pyrazolat (1c'), 60% Ma A und 20% der Verbindung Ir(DPBIC)₃ mit einer Dicke von 20 nm aufgedampft. 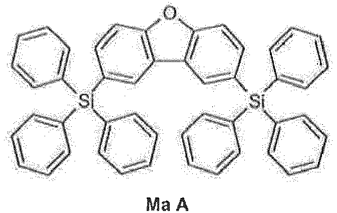 (zur Herstellung von Ma A siehe WO2009003898)

Anschließend wird Ma A mit einer Schichtdicke von 10nm als Loch- und ExcitonenBlocker aufgebracht. Als folgende Elektronenleiterschicht dient eine mit Cs₂CO₃ dotierte BCP Schicht mit einer Schichtdicke von 30nm. Eine Aluminiumkathode von 100nm Dicke schließt die Diode ab.

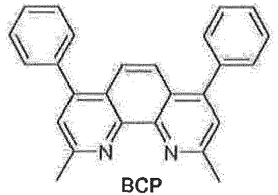

Alle Bauteile werden in einer inerten Stickstoffatmosphäre mit einem Glasdeckel verklebt.

### Diodenbeispiel 2:

Die auf die Lochinjektionsschicht folgende lichtemittierende Schicht wird ebenfalls aus Lösung aufgebracht. Hierzu wird eine Lösung mit 12.5 mg Feststoff pro 1 ml des Lösungsmittels Toluol hergestellt. Dabei besteht der Feststoffanteil aus 10% Di-[1-(Dibenzofuranyl)-3-methylimidazol-2-yliden-C2,C2']platin(II)di-pyrazolat (1a'), 30% der Matrix Ir(DPBIC)₃ und 60% der Ma A.

Nach der lichtemittierenden Schicht werden die nachfolgend genannten organischen Materialien mit einer Rate von ca. 0.5-5 nm/min bei etwa 10⁻⁷-10⁻⁹ mbar auf die lösungsprozessierten Schichten aufgedampft. Als Loch- und Excitonenblocker wird Ma A mit einer Dicke von 10 nm aufgebracht.

Als folgende Elektronenleiterschicht dient eine mit Cs₂CO₃ dotierte BCP Schicht mit einer Schichtdicke von 30nm. Eine Aluminiumkathode von 100nm Dicke schließt die Diode ab.

Alle Bauteile werden in einer inerten Stickstoffatmosphäre mit einem Glasdeckel verklebt.

Zur Charakterisierung des OLEDs werden Elektrolumineszenz-Spektren bei verschiedenen Strömen bzw. Spannungen aufgenommen. Weiterhin wird die Strom-Spannungs-Kennlinie in Kombination mit der abgestrahlten Lichtleistung gemessen. Die Lichtleistung kann durch Kalibrierung mit einem Luminanzmeter in photometrische Größen umgerechnet werden.
Für die beiden blauen Beispieldioden ergeben sich die folgenden elektrooptischen Daten:
Diodenbeispiel 1: CIE (0.17;0.21), maximale EQE* 8.3%
Diodenbeispiel 2: CIE (0.19, 0.34)

*EQE - externe Quanteneffizienz. Gemessen in Vorwärtsrichtung unter der Annahme einer Lambertschen Lichtintensitätsverteilung.

## Patentansprüche

1. Dinuklearer Pt-Carben-Komplex der allgemeinen Formel (1a) 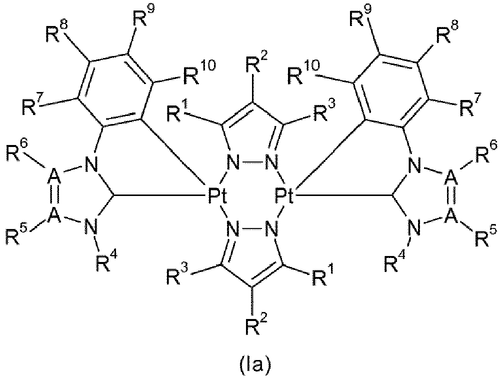 wobei A, R¹, R², R³, R⁴, R⁵, R⁶, R⁷, R⁸, R⁹ und R¹⁰ die folgenden Bedeutungen aufweisen:
A unabhängig voneinander N oder C;
R¹, R², R³ unabhängig voneinander Wasserstoff, linearer oder verzweigter, gegebenenfalls von wenigstens einem Heteroatom unterbrochener, gegebenenfalls wenigstens eine funktionelle Gruppe tragender Alkylrest mit 1 bis 10 Kohlenstoffatomen, Cycloalkylrest mit 3 bis 10 Kohlenstoffatomen;
R⁴ unabhängig voneinander, linearer oder verzweigter, gegebenenfalls von wenigstens einem Heteroatom unterbrochener, gegebenenfalls wenigstens eine funktionelle Gruppe tragender Alkylrest mit 1 bis 10 Kohlenstoffatomen, Cycloalkylrest mit 3 bis 10 Kohlenstoffatomen, substituierter oder unsubstituierter Arylrest mit 6 bis 18 Kohlenstoffatomen, substituierter oder unsubstituierter Heteroarylrest mit insgesamt 5 bis 18 Kohlenstoff- und/oder Heteroatomen;
R⁵, R⁶ unabhängig voneinander freies Elektronenpaar, falls A gleich N, oder, falls A gleich C, Wasserstoff, linearer oder verzweigter, gegebenenfalls von wenigstens einem Heteroatom unterbrochener, gegebenenfalls wenigstens eine funktionelle Gruppe tragender Alkylrest mit 1 bis 10 Kohlenstoffatomen, Cycloalkylrest mit 3 bis 10 Kohlenstoffatomen, substituierter oder unsubstituierter Arylrest mit 6 bis 18 Kohlenstoffatomen, substituierter oder unsubstituierter Heteroarylrest mit insgesamt 5 bis 18 Kohlenstoff- und/oder Heteroatomen,
oder
R⁴ und R⁵ oder R⁵ und R⁶ bilden zusammen mit N und A oder mit A und A einen gegebenenfalls von wenigstens einem weiteren Heteroatom unterbrochenen gesättigten, ungesättigten oder aromatischen Ring mit insgesamt 5 bis 18 Kohlenstoff- und/oder Heteroatomen;
R⁷, R⁸, R⁹, R¹⁰ unabhängig voneinander Wasserstoff, linearer oder verzweigter, gegebenenfalls von wenigstens einem Heteroatom unterbrochener, gegebenenfalls wenigstens eine funktionelle Gruppe tragender Alkylrest mit 1 bis 10 Kohlenstoffatomen, Cycloalkylrest mit 3 bis 10 Kohlenstoffatomen, substituierter oder unsubstituierter Arylrest mit 6 bis 18 Kohlenstoffatomen, substituierter oder unsubstituierter Heteroarylrest mit insgesamt 5 bis 18 Kohlenstoff- und/oder Heteroatomen, funktionelle Gruppe ausgewählt aus Ether-, Amino-, Thio-, Ester-, Carbonyl-, Nitro- oder Halogen-Gruppe,
oder
R⁷ und R⁸ oder R⁸ und R⁹ oder R⁹ und R¹⁰ bilden zusammen einen gegebenenfalls von wenigstens einem Heteroatom unterbrochenen gesättigten, ungesättigten oder aromatischen, gegebenenfalls substituierten, Ring mit insgesamt 5 bis 18 Kohlenstoff- und/oder Heteroatomen;
und/oder
R⁶ und R⁷ bilden zusammen eine gesättigte oder ungesättigte, lineare oder verzweigte, gegebenenfalls Heteroatome, aromatische Einheiten, heteroaromatische Einheiten und/oder funktionelle Gruppen enthaltende Verbrückung mit insgesamt 1 bis 30 Kohlenstoff- und/oder Heteroatomen, an die gegebenenfalls ein substituierter oder unsubstituierter, fünf bis achtgliedriger, Kohlenstoff- und/oder Heteroatome enthaltender Ring, annelliert ist.

2. Dinuklearer Pt-Carben-Komplex nach Anspruch 1, wobei R⁶ und R⁷ einen Phenylring entsprechend der Formel (IId) 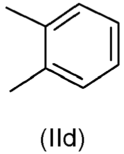 bilden.

3. Verfahren zur Herstellung der dinuklearen Pt-Carben-Komplexe gemäß einem der Ansprüche 1 oder 2 durch Inkontaktbringen von geeigneten Pt-Verbindungen mit den entsprechenden Liganden bzw. Ligandvorläufern und/oder Pyrazol bzw. entsprechenden Pyrazol-Derivaten.

4. Verfahren gemäß Anspruch 3, wobei geeignete Pt-Salze bzw. Pt-Komplexe mit geeigneten Ligandenvorläufern und Pyrazol bzw. entsprechenden Pyrazol-Derivaten umgesetzt werden,
wobei die Pt-Salze bzw. Pt-Komplexe vorzugsweise ausgewählt werden aus der Gruppe bestehend aus Pt(COD)Cl₂, Pt(PPh₃)₂Cl₂, Pt(Pyridin)₂Cl₂, Pt(Phenanthrolin)Cl₂, Pt(NH₃)₂CI₂, Pt(acac)₂, PtCl₂, K₂PtCI₄ und Mischungen davon, und
wobei die Ligandenvorläufer vorzugsweise der allgemeinen Formel (III) entsprechen: 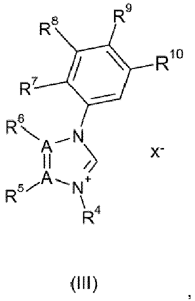 wobei R⁴, R⁵, R⁶, R⁷, R⁸, R⁹, R¹⁰ und A die gleichen Bedeutungen haben wie in Formel (Ia) und wobei X⁻ ausgewählt wird aus der Gruppe bestehend aus Halogenid, BF₄⁻, PF₆⁻, SbF₆⁻, ClO₄⁻, und SO₄²⁻.

5. Verfahren gemäß Anspruch 4, wobei die Ligandenvorläufer der allgemeinen Formel (IIIa) oder der allgemeinen Formel (IIIb) entsprechen:

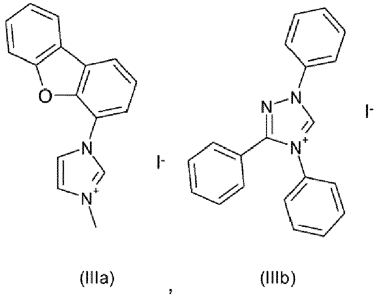

6. Verfahren gemäß einem der Ansprüche 3 bis 5 , wobei das Pyrazol bzw. die entsprechenden Pyrazol-Derivate ausgewählt werden aus den allgemeinen Formeln (IVa), (IVb), IVc), (IVd), (IVe), und (IVf):

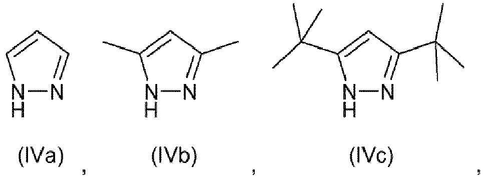

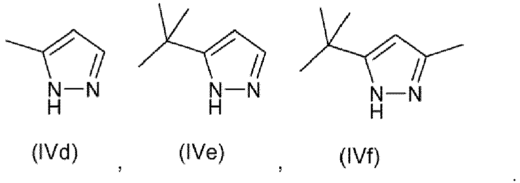

7. Verfahren gemäß Anspruch 4, wobei die Pt-Komplexe der allgemeinen Formel (V) entsprechen: 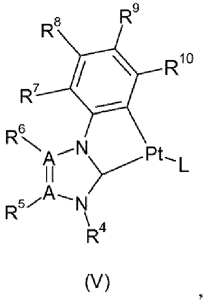 wobei R⁴, R⁵, R⁶, R⁷, R⁸, R⁹, R¹⁰ und A die gleichen Bedeutungen haben wie in Formel (Ia) und wobei L einen oder mehrere, ein- oder mehrzähnige Liganden darstellen, vorzugsweise Liganden ausgewählt aus der Gruppe bestehend aus cyclooctadien (cod), Cl, Br, Acetylacetonat (acac), 1,3-Diketiminate (nacnac), OAc (Acetat), BF₄, PF₆, PPh₃ oder Lösungsmittel.

8. Verfahren gemäß Anspruch 3, wobei Pt-Komplexe in denen der allgemeinen Formel (Ia) entsprechende Liganden vorliegen mit Pyrazol bzw. entsprechenden Pyrazol-Derivaten umgesetzt werden.

9. Organisches Elektronikbauteil, enthaltend wenigstens einen dinuklearen Pt-Carben-Komplex nach einem der Ansprüche 1 oder 2, wobei das organische Elektronikbauteil vorzugsweise ausgewählt wird aus der Gruppe bestehend aus Organische Licht-Emittierende Diode (OLED), Organische Photovoltaik Zelle (OPV), Organischer Feldeffekttransistor (OFET) oder Licht-Emittierende Elektrochemische Zelle (LEEC).

10. Licht-emittierende Schicht, enthaltend mindestens einen dinuklearen Pt-Carben-Komplex nach einem der Ansprüche 1 oder 2.

11. OLED, enthaltend eine Licht-emittierende Schicht nach Anspruch 10.

12. Vorrichtung ausgewählt aus der Gruppe bestehend aus stationären Bildschirmen, mobilen Bildschirmen und Beleuchtungsmitteln, enthaltend ein OLED gemäß Anspruch 9 oder 11.

13. Verwendung eines dinuklearen Pt-Carben-Komplexes nach einem der Ansprüche 1 oder 2 in OLEDs.

14. Verwendung nach Anspruch 13, **dadurch gekennzeichnet, dass** die dinuklearen Pt-Carben-Komplexe als Emitter, Matrixmaterial, Ladungstransportmaterial und/oder Ladungsblocker eingesetzt werden.

## Claims

1. A dinuclear Pt-carbene complex of the general formula (la) 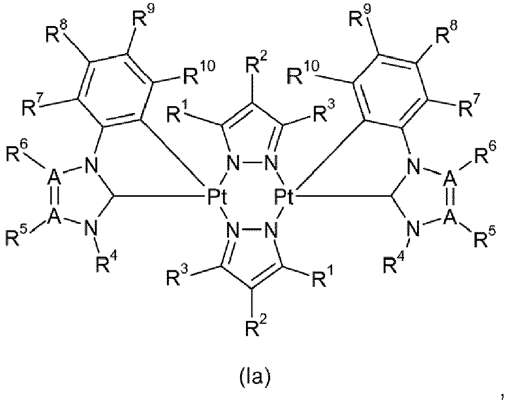 wherein A, R¹, R², R³, R⁴, R⁵, R⁶, R⁷, R⁸, R⁹ and R¹⁰ have the following meaning:
A is each independently N or C;
R¹, R², R³ is each independently hydrogen, a linear or branched alkyl radical having from 1 to 10 carbon atoms, optionally interrupted by at least one heteroatom, and optionally bearing at least one functional group, or a cycloalkyl radical having from 3 to 10 carbon atoms;
R⁴ is each independently a linear or branched alkyl radical having from 1 to 10 carbon atoms, optionally interrupted by at least one heteroatom, and optionally bearing at least one functional group, a cycloalkyl radical having from 3 to 10 carbon atoms, a substituted or unsubstituted aryl radical having from 6 to 18 carbon atoms, or a substituted or unsubstituted heteroaryl radical having a total of from 5 to 18 carbon atoms and/or heteroatoms;
R⁵, R⁶ is each independently a free electron pair if A is N, or, if A is C, hydrogen, a linear or branched alkyl radical having from 1 to 10 carbon atoms, optionally interrupted by at least one heteroatom, and optionally bearing at least one functional group, a cycloalkyl radical having from 3 to 10 carbon atoms, a substituted or unsubstituted aryl radical having from 6 to 18 carbon atoms, or a substituted or unsubstituted heteroaryl radical having a total of from 5 to 18 carbon atoms and/or heteroatoms,
or
R⁴ and R⁵, or R⁵ and R⁶ together with N and A, or together with A and A, form a saturated, or unsaturated, or aromatic ring having a total of from 5 to 18 carbon atoms and/or heteroatoms, and optionally interrupted by at least one heteroatom;
R⁷, R⁸, R⁹, R¹⁰ are each independently hydrogen, a linear or branched alkyl radical having from 1 to 10 carbon atoms, optionally interrupted by at least one heteroatom, and optionally bearing at least one functional group, a cycloalkyl radical having from 3 to 10 carbon atoms, a substituted or unsubstituted aryl radical having from 6 to 18 carbon atoms, a substituted or unsubstituted heteroaryl radical having a total of from 5 to 18 carbon atoms and/or heteroatoms, or a functional group selected from an ether group, an amino group, a thio group, an ester group, a carbonyl group, a nitro group, or a halogen group,
or
R⁷ and R⁸, or R⁸ and R⁹, or R⁹ and R¹⁰, together form a saturated , or unsaturated, or aromatic, optionally substituted ring having a total of from 5 to 18 carbon atoms and/or heteroatoms, optionally interrupted by at least one heteroatom;
and/or
R⁶ and R⁷ form a saturated or unsaturated, linear or branched bridge, optionally comprising heteroatoms, an aromatic unit, a heteroaromatic unit or functional groups, having a total of from 1 to 30 carbon atoms and/or heteroatoms, and optionally fused with a substituted or unsubstituted five- to eight-membered ring comprising carbon atoms and/or heteroatoms.

2. The dinuclear Pt-carbene complex of claim 1, wherein R⁶ and R⁷ form a phenyl ring according to formula (IId)

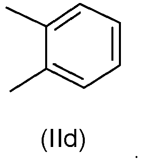

3. A process for preparing the dinuclear Pt-carbene complex according to any one of claims 1 or 2, by contacting suitable Pt compounds with a corresponding ligand or a corresponding ligand precursor, and/or a pyrazole or corresponding pyrazole derivatives.

4. The process of claim 3, wherein suitable Pt salts or Pt complexes are reacted with suitable ligand precursors and a pyrazole or pyrazole derivatives, wherein the Pt salts or Pt complexes are preferably selected from the group consisting of Pt(COD)Cl₂, Pt(PPh₃)₂Cl₂, Pt(pyridine)₂Cl₂, Pt(phenanthroline)Cl₂, Pt(NH₃)₂Cl₂, Pt(acac)₂, PtCl₂, K₂PtCI₄, and mixtures thereof, and
wherein the ligand precursors preferably correspond to the general formula (III): 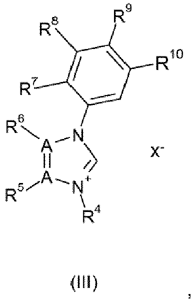 wherein R⁴, R⁵, R⁶, R⁷, R⁸, R⁹, R¹⁰ and A have the same meaning as in formula (Ia), and wherein X⁻ is selected from the group consisting of halogen, BF₄⁻, PF₆⁻, SbF₆⁻, ClO₄⁻, and SO₄²⁻.

5. The process of claim 4, wherein the ligand precursors correspond to the general formula (IIIa) or to the general formula (IIIb):

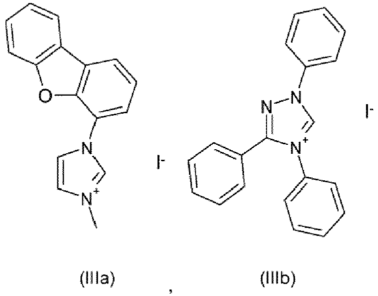

6. The process of any one of claims 3 to 5, wherein the pyrazole or the corresponding pyrazole derivatives are selected from the general formulae (IVa), (IVb), IVc), (IVd), (IVe), and (IVf):

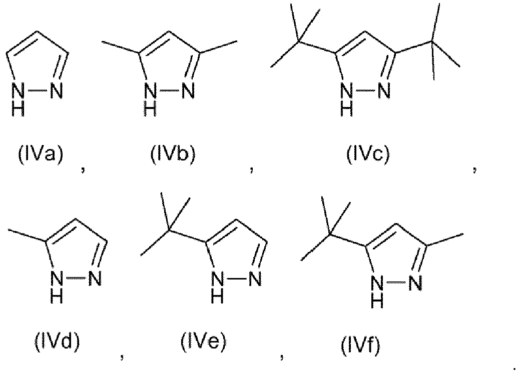

7. The process of claim 4, wherein the Pt complex correspond to the general formula (V): 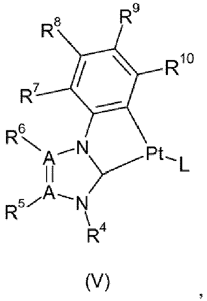 wherein R⁴, R⁵, R⁶, R⁷, R⁸, R⁹, R¹⁰ and A have the same meanings as in formula (Ia), and wherein the L is/are one or more mono- or multidentate ligands, preferably ligands selected from the group consisting of cyclooctadiene (cod), Cl, Br, acetyl acetonate (acac), 1,3-diketiminate (nacnac), OAc (acetate), BF₄, PF₆, PPh₃ or a solvent.

8. The process of claim 3, wherein Pt complexes having ligands corresponding to general formula (Ia) are reacted with pyrazole or corresponding pyrazole derivatives.

9. An organic electronic component, comprising at least one dinuclear Pt-carbene complex according to one of claims 1 or 2, wherein the organic electronic component is preferably selected from the group consisting of organic light-emitting diodes (OLED), organic photovoltaic cells (OPV), organic field effect transistors (OFET), or light-emitting electrochemical cells (LEEC).

10. A light-emitting layer, comprising at least one dinuclear Pt-carbene complex according to one of claims 1 or 2.

11. An OLED, comprising a light-emitting layer according to claim 10.

12. A device selected from the group consisting of stationary displays, mobile displays, and illumination devices, comprising an OLED according to claim 9 or 11.

13. A use of a dinuclear Pt-carbene complex according to one of claims 1 or 2 in OLEDs.

14. The use of claim 13, **characterized in that** the dinuclear Pt-carbene complexes are utilized as an emitter, a matrix material, a charge carrying material and/or a charge blocking material.

## Revendications

1. Complexe Pt-carbène binucléaire de formule générale (la) : 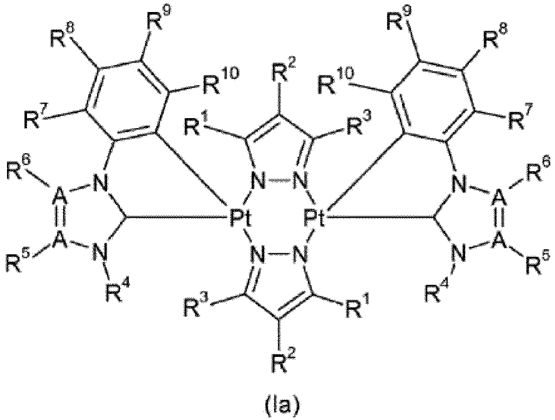
A, R¹, R², R³, R⁴, R⁵, R⁶, R⁷, R⁸, R⁹ et R¹⁰ ayant les significations suivantes :
A chacun indépendamment N ou C,
R¹, R², R³ chacun indépendamment un hydrogène, un radical alkyle linéaire ou ramifié possédant 1 à 10 atomes de carbone, éventuellement interrompu par au moins un hétéroatome, portant éventuellement au moins un groupe fonctionnel, un radical cycloalkyle possédant 3 à 10 atomes de carbone,
R⁴ chacun indépendamment un radical alkyle linéaire ou ramifié possédant 1 à 10 atomes de carbone, éventuellement interrompu par au moins un hétéroatome, portant éventuellement au moins un groupe fonctionnel, un radical cycloalkyle possédant 3 à 10 atomes de carbone, un radical aryle substitué ou non substitué possédant 6 à 18 atomes de carbone, un radical hétéroaryle substitué ou non substitué possédant au total 5 à 18 atomes de carbone et/ou hétéroatomes,
R⁵, R⁶ chacun indépendamment un doublet électronique libre, si A égale N, ou, si A égale C, un hydrogène, un radical alkyle linéaire ou ramifié possédant 1 à 10 atomes de carbone, éventuellement interrompu par au moins un hétéroatome, portant éventuellement au moins un groupe fonctionnel, un radical cycloalkyle possédant 3 à 10 atomes de carbone, un radical aryle substitué ou non substitué possédant 6 à 18 atomes de carbone, un radical hétéroaryle substitué ou non substitué possédant au total 5 à 18 atomes de carbone et/ou hétéroatomes,
ou
R⁴ et R⁵ ou R⁵ et R⁶ forment conjointement avec N et A ou avec A et A un composé cyclique saturé, insaturé ou aromatique, éventuellement interrompu par au moins un autre hétéroatome, possédant au total 5 à 18 atomes de carbone et/ou hétéroatomes,
R⁷, R⁸, R⁹, R¹⁰ chacun indépendamment un hydrogène, un radical alkyle linéaire ou ramifié possédant 1 à 10 atomes de carbone, éventuellement interrompu par au moins un hétéroatome, portant éventuellement au moins un groupe fonctionnel, un radical cycloalkyle possédant 3 à 10 atomes de carbone, un radical aryle substitué ou non substitué possédant 6 à 18 atomes de carbone, un radical hétéroaryle substitué ou non substitué possédant au total 5 à 18 atomes de carbone et/ou hétéroatomes, un groupe fonctionnel choisi parmi le groupe éther, amino, thio, ester, carbonyle, nitro ou halogène,
ou
R⁷ et R⁸ ou R⁸ et R⁹ ou R⁹ et R¹⁰ forment conjointement un composé cyclique saturé, insaturé ou aromatique, éventuellement substitué, éventuellement interrompu par au moins un hétéroatome, possédant au total 5 à 18 atomes de carbone et/ou hétéroatomes,
et/ou
R⁶ et R⁷ forment conjointement un pont saturé ou insaturé, linéaire ou ramifié, contenant éventuellement des hétéroatomes, des unités aromatiques, des unités hétéroaromatiques et/ou des groupes fonctionnels, possédant au total 1 à 30 atomes de carbone et/ou hétéroatomes, auquel un composé cyclique substitué ou non substitué, à cinq à huit chaînons, contenant des atomes de carbone et/ou des hétéroatomes, est éventuellement condensé.

2. Complexe Pt-carbène binucléaire selon la revendication 1, dans lequel R⁶ et R⁷ forment un cycle phényle correspondant à la formule (IId)

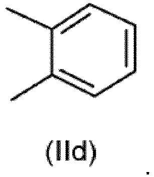

3. Procédé de préparation des complexes Pt-carbène binucléaires selon l'une des revendications 1 ou 2, par mise en contact de liaisons Pt appropriées avec les ligands ou précurseurs de ligands correspondants et/ou du pyrazole ou des dérivés de pyrazole correspondants.

4. Procédé selon la revendication 3, dans lequel des sels Pt ou des complexes Pt appropriés réagissent avec des précurseurs de ligands appropriés et du pyrazole ou des dérivés de pyrazole correspondants,
dans lequel les sels Pt ou les complexes Pt sont de préférence choisis parmi le groupe constitué de Pt(COD)Cl₂, Pt(PPh₃)₂Cl₂, Pt(pyridine)₂Cl₂, Pt(phénanthroline)Cl₂, Pt(NH₃)₂Cl₂, Pt(acac)₂, PtCl₂, K₂PtCl₄ et des mélanges de ceux-ci, et
dans lequel les précurseurs de ligands correspondent de préférence à la formule générale (III) : 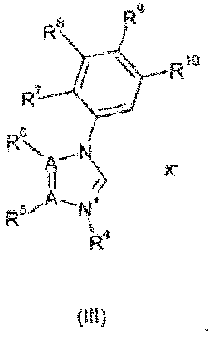 dans laquelle R⁴, R⁵, R⁶, R⁷, R⁸, R⁹, R¹⁰ et A ont les mêmes significations que dans la formule (la) et dans laquelle X⁻ est choisi parmi le groupe constitué d'un halogénure, de BF₄⁻, PF₆⁻, SbF₆⁻, ClO₄⁻ et SO₄²⁻.

5. Procédé selon la revendication 4, dans lequel les précurseurs de ligands correspondent à la formule générale (IIIa) ou à la formule générale (IIIb) :

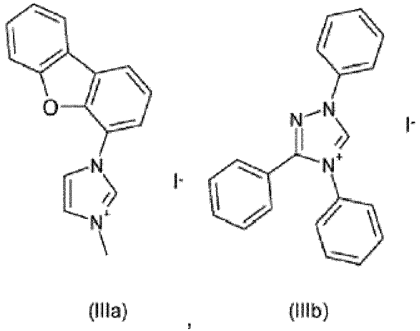

6. Procédé selon l'une des revendications 3 à 5, dans lequel le pyrazole ou les dérivés de pyrazole correspondants sont choisis parmi les formules générales (IVa), (IVb), (IVc), (IVd), (IVe) et (IVf) :

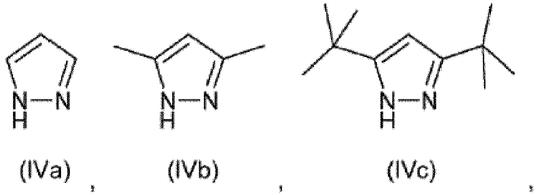

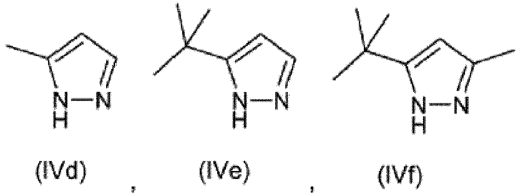

7. Procédé selon la revendication 4, dans lequel les complexes Pt correspondent à la formule générale (V) : 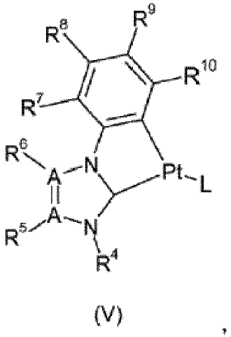 dans laquelle R⁴, R⁵, R⁶, R⁷, R⁸, R⁹, R¹⁰ et A ont les mêmes significations que dans la formule (la) et dans laquelle L représente un ou plusieurs ligands monodentés ou pluridentés, de préférence des ligands choisis parmi le groupe constitué de cyclooctadiène (cod), Cl, Br, acétylacétonate (acac), 1,3-dicétiminate (nacnac), OAc (acétate), BF₄, PF₆, PPh₃ ou de solvant.

8. Procédé selon la revendication 3, dans lequel des complexes Pt, dans lesquels des ligands correspondant à la formule générale (la) sont présents, réagissent avec du pyrazole ou des dérivés de pyrazole correspondants.

9. Composant électronique organique, contenant au moins un complexe Pt-carbène binucléaire selon l'une des revendications 1 ou 2, dans lequel le composant électronique organique est de préférence choisi parmi le groupe constitué d'une diode électroluminescente organique (OLED), d'une cellule photovoltaïque organique (OPV), d'un transistor à effet de champ organique (OFET) ou d'une cellule électrochimique émettrice de lumière (LEEC).

10. Couche émettrice de lumière, contenant au moins un complexe Pt-carbène binucléaire selon l'une des revendications 1 ou 2.

11. OLED, contenant une couche émettrice de lumière selon la revendication 10.

12. Dispositif choisi parmi le groupe constitué d'écrans fixes, d'écrans mobiles et de moyens d'éclairage, contenant une OLED selon l'une des revendications 9 ou 11.

13. Utilisation d'un complexe Pt-carbène binucléaire selon l'une des revendications 1 ou 2 dans des OLED.

14. Utilisation selon la revendication 13, **caractérisée en ce que** les complexes Pt-carbène binucléaires sont utilisés en tant qu'émetteurs, matériau de matrice, matériau de transport de charge et/ou bloqueurs de charge.
